# EUROPEAN PATENT SPECIFICATION

(11) **EP 0 910 662 B1**
(45) Date of publication and mention of the grant of the patent: **08.11.2000**
(21) Application number: 96907091.1
(22) Date of filing: 20.02.1996
(51) Int. Cl.: C12Q 1/68, C07H 21/04, C12P 19/34

(54) **METHODS AND REAGENTS FOR TYPING HLA CLASS I GENES**
VERFAHREN UND REAGENZIEN ZUR TYPISIERUNG VON HLA-GENEN DER KLASSE I
PROCEDE ET REACTIFS DE TYPAGE DE GENES HLA DE CLASSE I

(43) Date of publication of application: 28.04.1999
(73) Proprietor: THE PERKIN-ELMER CORPORATION, Foster City, California 94404 (US)
(72) Inventor: JOHNSTON-DOW, Leslie, Palo Alto, CA 94308 (US); CHADWICK, Robert, B., Hayward, CA 94554 (US); PARHAM, Peter, Stanford, CA 94305 (US)
(74) Representative: Vossius, Volker, Dr.
(86) International application number: US9602408
(87) International publication number: WO9731126

(56) References cited:
- EP-A- 0 414 469
- EP-A- 0 540 997
- WO-A-92/07956
- WO-A-92/19771
- WO-A-94/21818
- TISSUE ANTIGENS, vol. 45, no. 4, April 1995, pages 223-31, XP000573595 KRAUSA P ET AL: "Genetic polymorphism within HLA-A*02: significant allelic variation revealed in different populations"
- TISSUE ANTIGENS, vol. 46, no. 2, August 1995, pages 86-95, XP000573581 BLASCZYK R ET AL: "Complete subtyping of the HLA-A locus by direct sequencing or single strand conformation polymorphism analysis"
- TISSUE ANTIGENS, vol. 46, September 1995, pages 187-95, XP000573587 KENNEDY L ET AL: "Definition of HLA-C alleles using sequence specific oligonucleotide probes"

## Description

### BACKGROUND

The present invention relates to methods and reagents for typing HLA class I genes utilizing locus-specific nucleic acid amplification followed by sequence-specific detection of the amplified product.

The immune system has evolved a special mechanism to detect infections which occur within cells as opposed to infections which occur in extracellular fluid or blood, e.g., Janeway Jr., Scientific American, September, 73-79 (1993). Generally speaking, this mechanism acts in two steps: first, the immune system finds a way to signal to the body that certain cells have been infected, and then, it mobilizes cells specifically designed to recognize these infected cells and to eliminate the infection.

The initial step, signaling that a cell is infected, is accomplished by special molecules that deliver segments of the invading microbe to the outer surface of the infected cell. These molecules bind to peptide fragments of the invading microbe and then transport the peptides to the outside surface of the infected cell.

These transporter molecules are proteins of the major histocompatibility complex of genes, which in humans is referred to as the human leukocyte antigen complex, or HLA. These HLA molecules can be divided into two Classes: (i) class I molecules which are found on almost all types of cells, and (ii) class II molecules which appear only on cells involved in the immune response.

The two different classes of HLA molecules present peptides that arise in different places within cells. Class I molecules bind to peptides that originate from proteins in the cystolic compartment of the cell. After binding, the class I molecules fold around the foreign peptide then carry the peptide to the cell surface. The presentation of the peptide by the class I molecules then signals other cells of the immune system to destroy the host cell. The genes coding for the class I molecules are further subclassified as the HLA-A, HLA-B, and HLA-C genes.

Unlike class I molecules, class II molecules do not require peptide-directed folding to become active. Moreover, rather than signaling the destruction of the host cell, peptides presented by the class II molecules activate the internal defenses of the host cell, or alternatively, guide the synthesis of specific antibody molecules by the immune system.

The genes that encode the HLA molecules are among the most variable genes in humans: each variant coding for molecules which bind to different peptides. These genes are the same in all the cells of a particular individual, but differ from person to person.

HLA typing is performed routinely in connection with many medical indications, e.g., organ transplantation (rejection of organ grafts is believed to be greatly diminished if the HLA alleles of donor and recipient are identical), the study of auto-immune disease, and the determination of susceptibility to infectious disease.

Traditionally, the majority of HLA typing has been performed using serological techniques. However, these techniques have a number of serious drawbacks: (i) the availability of standard antisera is limited, (ii) the accuracy and resolution of the technique is limited by the small number of alleles which can be tested for, (iii) the speed of serological tests is very slow, and, (iv) new alleles can not be detected.

Recently, to solve many of the problems of serologically-based typing methods, molecular techniques have been employed for HLA typing, including restriction fragment length polymorphism analysis (RFLP), sequence specific oligonucleotide probing and/or priming techniques, and DNA sequencing. By looking directly at the genotype of the HLA system rather than the phenotype, the information content and accuracy of the typing procedure can be greatly enhanced. Examples of sequencingbased methods are provided by Santamaria et al., WO-A-9 215 711; Holtz et al., DNA-Technology and Its Forensic Application, Berghaus et al. eds., p 79-84 (1991); Petersdorf et al., Tissue Antigens 44: 211-216 (1994); Guttridge et al., Tissue Antigens 44; 43-46 (1994); Santamaria et al., Human Immunology 37: 39-50 (1993); Santamaria et al., Human Immunology 33: 69-81 (1992); and Petersdorf et al., Tissue Antigens, 43: in press (1994). Examples of probing-based methods are provided by Bunce and Welsh, Tissue Antigens 43: 7-17 (1994); Anrien et al., Tissue Antigens 42: 480-487 (1993); Dominguez et aL, Immunogenetics, 36: 277-282 (1992); Yoshida et al., Human Immunology 34: 257-266 (1992); Allen et al., Human Immunology 40: 25-32 (1994); Fernandez-Vina et al., Human Immunology 33:163-173 (1992); and Teodorica and Erlich, EPO EP-A-540997 Santamaria et al.; WO 92/19771 disclose a process for determining Class I HLA genotypes of exons 2, 3 and 4 by amplification of cDNA or genomic DNA and sequencing of the amplified DNA.

Important problems encountered in any of the above molecular techniques include the complexity, reliability and specificity of the DNA amplification procedures. These problems have become particularly critical as these molecular typing techniques have become more commonly used in the clinical environment. Because of the similarity among the HLA-A, -B, and -C genes, it has been up until very recently impossible to find amplification methods which allow discrimination between the three class I genes while at the same time are independent of (i) inadvertent amplification of closely related genes, e.g., neighboring pseudogenes, and (ii) independent of the extreme polymorphism found in these genes, e.g., Cereb et aL, Tissue Antigens 45: 1-11 (1995). However, the protocol of Cereb relies on intronic primer sites, making it susceptible to promiscuous intronic mutations.

Current techniques have addressed these problems by limiting the generality of the methods, e.g., limiting the analysis to a subset of exons in a given gene, e.g., Petersdorfet al., Tissue Antigens, 44: 93-99 (1994), where the analysis requires multiple amplification primer sets and sequential amplifications to cover only the HLA-C subtype. This approach is not preferred because of the complexity of the protocols, the amount of information about the sample required prior to the analysis, and the number of reagents required.

### SUMMARY

The present invention relates to our discovery of methods and reagents for the DNA typing of HLA class I genes utilizing locus-specific nucleic acid amplification followed by sequence-specific detection of the amplified product.

An object of our invention is to provide methods and reagents for the amplification of the HLA-A, HLA-B, and HLA-C genes of the HLA class I gene family wherein the amplification is able to discriminate among the HLA-A, HLA-B, and HLA-C genes and other related class I genes and pseudogenes.

A further object of our invention is to provide methods and reagents for the specific amplification of the HLA-A, HLA-B, and HLA-C genes of the HLA class I gene family which are not subject to variability due to intronic sequence polymorphisms.

An additional object of our invention is to provide methods and reagents for the specific amplification of each of the HLA-A, HLA-B, and HLA-C genes of the HLA class I gene family wherein a single set of amplification primers serve to amplify the informative regions of each of the HLA-A, -B, or -C genes.

Another object of our invention is to provide methods and reagents for the specific DNA sequencing of the HLA-A, HLA-B, and HLA-C genes of the HLA class I gene family wherein specificity is maximized while the number of sequencing primers and method steps is minimized.

Still another object of our invention is to provide various reagent kits useful for the practice of the aforementioned methods.

Another object of our invention is to provide methods and reagents for the specific DNA sequencing of the HLA-A, HLA-B, and HLA-C genes of the HLA class I gene family wherein the sample DNA is genomic DNA.

The foregoing and other objects of the invention are achieved by a method for typing HLA class I genes wherein a sample DNA containing a HLA class I gene is contacted with a first amplification primer, the first primer including sequence complementary to a first exon of a HLA class I gene, and a second amplification primer, the second primer including sequence complementary to a second exon of the HLA class I gene, wherein the first and second primers are complementary to opposite strands of the sample DNA. Then, a portion of the sample DNA is amplified by PCR using the first and second primers. Finally, the amplicon formed by the PCR is detected using a sequence-specific detection method.

In a preferred embodiment of the present invention, the sequence-specific detection method is DNA sequencing.

In another aspect, the present invention provides amplification primers and sequencing primers adapted for carrying out the above HLA typing method.

In yet another aspect, the present invention provides kits for carrying out the above HLA typing method.

These and other objects, features, and advantages of the present invention will become better understood with reference to the following description, appended claims, and drawings.

### BRIEF DESCRIPTION OF THE DRAWINGS

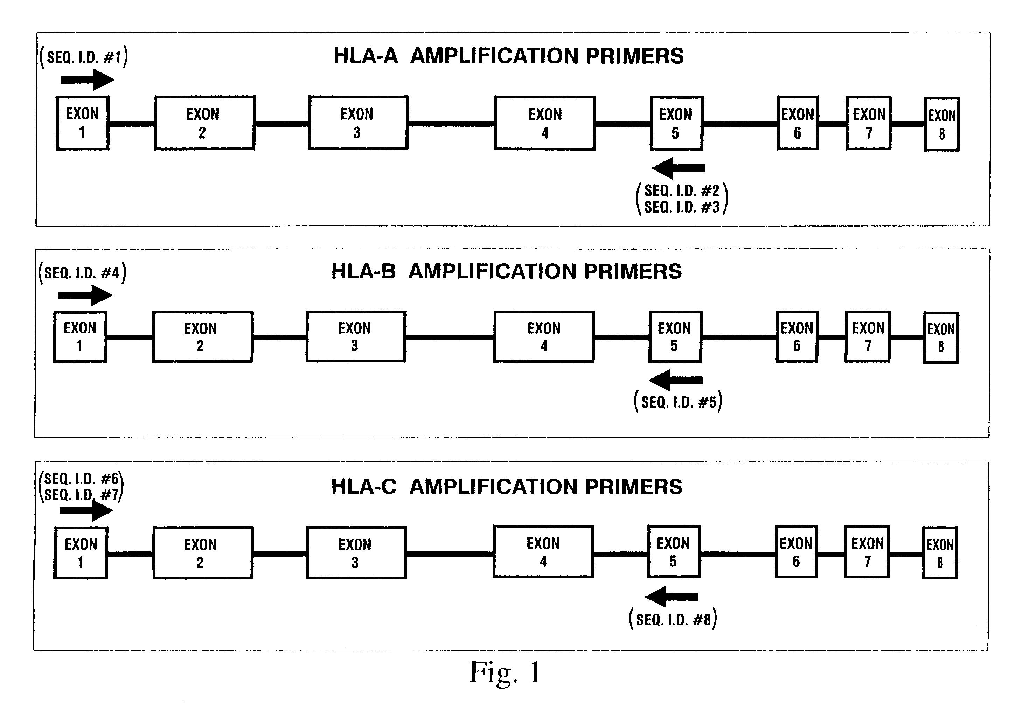

Fig. 1 is a schematic map of the amplification primer locations.

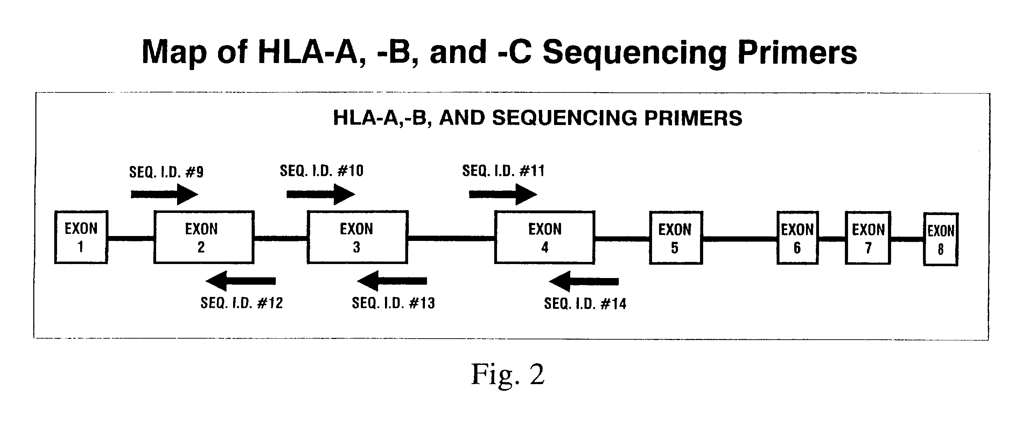

Fig. 2 is a schematic map of the sequencing primer locations.

Fig. 3 is the raw sequence of the sense strand of exon 2 of the HLA-A gene.

Fig. 4 is the raw sequence of the antisense strand of exon 2 of the HLA-A gene.

Fig. 5 is the sequence alignment of the sequences of Figs. 3 and 4.

Fig. 6 is the raw sequence of the sense strand of exon 3 of the HLA-A gene.

Fig. 7 is the raw sequence of the antisense strand of exon 3 of the HLA-A gene.

Fig. 8 is the sequence alignment of the sequences of Figs. 6 and 7.

Fig. 9 is the raw sequence of the sense strand of exon 2 of the HLA-B gene.

Fig. 10 is the raw sequence of the antisense strand of exon 2 of the HLA-B gene.

Fig. 11 is the sequence alignment of the sequences of Figs. 9 and 10.

Fig. 12 is the raw sequence of the antisense strand of exon 3 of the HLA-B gene.

Fig. 13 is the sequence alignment of the sequence of Fig. 12.

Fig. 14 is the raw sequence of the sense strand of exon 4 of the HLA-B gene.

Fig. 15 is the raw sequence of the antisense strand of exon 4 of the HLA-B gene.

Fig. 16 is the sequence alignment of the sequences of Figs. 14 and 15.

Fig. 17 is the raw sequence of the sense strand of exon 2 of the HLA-C gene.

Fig. 18 is the raw sequence of the antisense strand of exon 2 of the HLA-C gene.

Fig. 19 is the sequence alignment of the sequences of Figs. 17 and 18.

Fig. 20 is the raw sequence of the antisense strand of exon 3 of the HLA-C gene.

Fig. 21 is the sequence alignment of the sequences of Fig. 20.

Fig. 22 is the raw sequence of the sense strand of exon 2 of the HLA-A gene.

Fig. 23 is the raw sequence of the antisense strand of exon 2 of the HLA-A gene.

Fig. 24 is the sequence alignment of the sequences of Figs. 22 and 23.

Fig. 25 is the raw sequence of the sense strand of exon 3 of the HLA-A gene.

Fig. 26 is the raw sequence of the antisense strand of exon 3 of the HLA-A gene.

Fig. 27 is the sequence alignment of the sequences of Figs. 25 and 26.

Fig. 28 is the raw sequence of the sense strand of exon 2 of the HLA-B gene.

Fig. 29 is the raw sequence of the antisense strand of exon 2 of the HLA-B gene.

Fig. 30 is the sequence alignment of the sequences of Figs. 28 and 29.

Fig. 31 is the raw sequence of the sense strand of exon 3 of the HLA-B gene.

Fig. 32 is the raw sequence of the antisense strand of exon 3 of the HLA-B gene.

Fig. 33 is the sequence alignment of the sequences of Figs. 31 and 32.

Fig. 34 is the raw sequence of the sense strand of exon 2 of the HLA-C gene.

Fig. 35 is the raw sequence of the antisense strand of exon 2 of the HLA-C gene.

Figs. 36A and 36B are the sequence alignment of the sequences of Figs. 34 and 35.

Fig. 37 is the raw sequence of the antisense strand of exon 3 of the HLA-C gene.

Fig. 38 is the sequence alignment of the sequence of Fig. 37.

### DESCRIPTION OF THE PREFERRED EMBODIMENTS

The present invention is directed to methods and reagents for typing HLA class I genes utilizing locus-specific nucleic acid amplification followed by sequence-specific detection of the amplified product. Generally, the method of the present invention involves amplification of a segment of one or a combination of the HLA-A, HLA-B, and/or HLA-C genes, e.g. by the polymerase chain reaction (PCR), followed by detection of the resulting amplicon(s) by a detection method capable of distinguishing between different-sequence amplicons, e.g., DNA sequencing, sequence specific oligonucleotide probes, restriction digestion, and the like. The reagents of the instant invention include preferred PCR primers and preferred sequencing primers.

### 1. Definitions

As used herein, the term "gene" refers to a segment of DNA composed of a transcribed region and a regulatory sequence that makes possible a transcription, including both the sense and antisense strands of the DNA. The terms "locus" or "gene locus" refers to the specific place on a chromosome where a gene is located. The term "allele" refers to the multiple forms of a gene that can exist at a single gene locus.

As used herein, the terms "HLA class I genes" or "HLA class I gene family" refer to a subgroup of the genes of the human leukocyte antigen complex located on the short arm of chromosome 6 in the distal portion of the 6p21.3 chromosome, the other members of the human leukocyte antigen complex being the class II and class III genes, e.g., Trowsdale et al., Immunology Today, 12(12): 443-446 (1991).

As used herein, the term "exon" refers to a polynucleotide segment that codes for amino acids, while those segments that are not translated into amino acids are referred to herein as "introns". The term "intron-exon border" refers the interface of the intron segment and the exon segment. When referring to the two strands making up a double stranded DNA molecule, as used herein, the term "antisense strand" or "minus strand" refers to the strand of the pair which serves as the template for transcription of DNA into mRNA, while the term "sense strand" or "plus strand" refers to the other strand of the pair which carries the codons for translation in its sequence.

The term "oligonucleotide" or "polynucleotide" as used herein refer to a molecule having two or more deoxyribonucleotides or ribonucleotides, the number of nucleotides in the molecule depending on its intended function. As used herein the term "primer" refers to an oligonucleotide, preferably produced synthetically, which, when hybridized to a complementary template strand of DNA, is capable of acting as a point of initiation for synthesis of a primer extension product. The terms "first amplification primer" and "second amplification primer" refer to members of a pair of PCR primers which are used to initiate amplification of a "target sequence" of the sample DNA. The first primer and second primer are complementary to opposite strands of the target DNA, i.e., antisense and sense strands, and are located at the opposite ends of the target sequence. A "set" of first primers or second primers refer to a collection of two or more degenerate primers complementary to multiple alleles located at the same gene locus, where, as used herein, the term "degenerate primers" refers to a collection of primers differing in sequence by base substitutions at one or more particular sequence locations.

### 2. Oligonucleotide Primers

The oligonucleotide primers, both sequencing primers and amplification primers, can be synthesized using any suitable method, e.g., phosphoramidite, phosphotriester, phosphite-triester or phosphodiester synthesis methods, e.g., Gait, Oligonucleotide Synthesis, IRL Press, Washington, DC (1984). Preferably, the oligonucleotides of the present invention are prepared using an automated DNA synthesizer, e.g., Applied Biosystems Model 392 DNA Synthesizer (Applied Biosystems Division of the Perkin-Elmer Corporation (ABI), Foster City, CA). In a less preferred method, primers can be isolated from a biological source using appropriate restriction endonucleases.

### 3. Preparation of Sample DNA

Any source of human nucleic acid can be used to obtain the sample nucleic acid as long as the source contains the nucleic acid sequence of interest. Typical samples include peripheral blood mononuclear cells (PBMNCs), lymphoblastoid cell lines (LCLs), hair cells, or the like. Preferably genomic DNA extracted from PBMNCs or LCLs is used.

A large number of methods are available for the isolation and purification of sample DNA for use in the present invention. The preferred purification method should provide sample DNA (i) sufficiently free of protein to allow efficient nucleic acid amplification and sequencing and (ii) of a size sufficient to allow trans-intronic amplification of the class I genes. Preferred purification methods include (i) organic extraction followed by ethanol precipitation, e.g., using a phenol/chloroform organic reagent, e.g., Ausubel et al. eds., Current Protocols in Molecular Biology Volume 1, Chapter 2, Section 1, John Wiley & Sons, New York, NY (1993), preferably using an automated DNA extractor, e.g., the Model 341 DNA Extractor availahle from ABI; (ii) solid phase adsorption methods, e.g., Walsh et al., Biotechniques 10(4): 506-513 (1991); and (iii) salt-induced DNA precipitation methods, e.g., Miller et al., Nucleic Acids Research 16(3): 9-10 (1988), such methods being typically referred to as 'salting-out" methods. More preferably, the sample DNA is purified by salt-induced DNA precipitation methods. Preferably, each of the above purification methods is preceded by an enzyme digestion step to help eliminate protein from the sample, e.g., digestion with proteinase K, or like proteases.

### 4. PCR Amplification

Amplification of sample DNA for each gene locus of interest is accomplished using the polymerase chain reaction (PCR) as generally described in U.S. Pat. Nos. 4,683,195 and 4,683,202 to Mullis. Generally, the PCR consists of an initial denaturation step which separates the strands of a double stranded nucleic acid sample, followed by the repetition of (i) an annealing step, which allows amplification primers to anneal specifically to a portion of a target sequence of the separated strands of the sample DNA molecule or copies thereof; (ii) an extension step which extends the primers in a 5' to 3' direction thereby forming an amplicon nucleic acid complementary to the target sequence, and (iii) a denaturation step which causes the separation of the amplicon and the target sequence. Each of the above steps may be conducted at a different temperature, where the temperature changes may be accomplished using a thermocycler (Perkin-Elmer Corporation, Norwalk, CT (PE)).

The present invention introduces the use of locus-specific amplification primers including sequence which is complementary to conserved exon sequences, where, in a preferred embodiment, the conserved exon sequences are not involved in antigenic determination, but differ in sequence between related genes, e.g., between HLA-A, -B, and -C genes, and related pseudogenes. In contrast to existing HLA typing methods which include primers complementary to purely intronic sequence, the primers of the present invention provide a number of critically important practical advantages.

One important advantage of the present invention is that it makes possible the amplification of the entire informative region of a class I gene in a single PCR product amplicon. As used herein, the term "informative region" refers to a region of a class I gene which is polymorphic between different individuals and codes for portions of the class I proteins which are involved with antigenic determination. By enabling the amplification of the entire informative region in one amplicon, the method of the present invention requires fewer amplification primers, preferably only one pair per gene, leading to the use of fewer reaction tubes and fewer steps in the amplification protocol.

A second significant advantage of the amplification aspect of HLA typing method of the present invention is that the preferred amplification primers can distinguish between the class I genes, i.e., HLA-A, HLA-B, and HLA-C genes, and all closely related genes and pseudogenes, where, as used herein, the term "pseudogene" refers to neighboring sequence regions which are highly homologous to a particular expressed gene but does not give rise to detectable levels of mRNA. This ability to distinguish between related genes is a particularly import feature of the present invention given the extreme level of polymorphism between genes of different individuals and the similar structure of the HLA class I genes.

By building the gene-level specificity of the typing procedure into the amplification step, the post-amplification detection steps do not require gene-level specificity. Again, this significantly reduces the complexity of the overall typing method. For example, when DNA sequencing is used as the detection step (see below), one set of sequencing primers can be used for sequencing all the class I genes, resulting in many fewer sequencing primers and fewer sequencing reactions.

A further considerable advantage of the amplification step of the present invention is that the quality of the amplification is not subject to intronic polymorphisms. This provides for a much more reproducible and robust technique which is immune to the extreme variability of these regions.

Yet another advantage of the amplification step of the present invention is that the sample DNA that is used as the amplification template is genomic DNA rather than mRNA. This makes the amplification process independent of the varying amounts of mRNA present in sample donors depending on their medical condition, e.g., their degree of immuno suppression, greatly enhancing the reliability of the present typing method. Furthermore, because of the poor chemical stability of mRNA, using genomic target DNA reduces the demand placed on the preparation and storage of the sample material.

In a preferred embodiment, the amplification primers are between five nucleotides and 50 nucleotides in length; more preferably between 10 nucleotides and 30 nucleotides in length. This size range provides primers long enough to have a statistically unique sequence, thereby resulting in high stringency hybridization, and short enough to allow easy synthesis and favorable hybridization kinetics.

For the analysis of the HLA-A class I genes, the preferred amplification primers are chosen such that the first amplification primer is complementary to sequence located in exon 1 of the HLA-A gene, and the second amplification primer is complementary to sequence located in exon 5 of the HLA-A gene. Preferably, the first amplification primer comprises a set of degenerate primers having between one and three degenerate sequence locations. More preferably, the first amplification primer includes the sequence CGCCGAGGATGGCCGTC (SEQ ID #1) and the second amplification primer comprises a set of two degenerate primers, the first degenerate primer of the set including the sequence GGAGAACCAGGCCAGCAATGATGCCC (SEQ ID #2), and the second degenerate primer of the set including the sequence GGAGAACTAGGCCAGCAATGATGCCC (SEQ ID #3), where the underlined nucleotides indicates the location of the C→ T degeneracy (note that all sequences reported herein are written in a 5' to 3' orientation). In a more preferred embodiment, the first degenerate primer (SEQ ID #2) and the second degenerate primer (SEQ ID #3) are present in a 1:1 molar ratio.

For the analysis of the HLA-B class I genes, the preferred amplification primers are chosen such that the first amplification primer is complementary to sequence located in exon 1 of the HLA-B gene, and the second amplification primer is complementary to sequence located in exon 5 of the HLA-B gene. Preferably, the first amplification primer includes the sequence GGCCCTGACCGAGACCTGG (SEQ ID #4) and the second amplification primer includes the sequence TCCGATGACCACAACTGCTAGGAC (SEQ ID #5). More preferably, the first amplification primer includes the sequences CCTCCTGCTGCTCTCGGC (SEQ ID #21), CCTCCTGCTGCTCTCGGGA (SEQ ID #22), and GCTGCTCTGGGGGGCAG (SEQ ID #23), and the second amplification primer includes the sequence GCTCCGATGACCACAACTGCT (SEQ ID #24).

For the analysis of the HLA-C class I genes, the preferred amplification primers are chosen such that the first amplification primer is complementary to sequence located in exon 1 of the HLA-C gene, and the second amplification primer is complementary to sequence located in exon 5 of the HLA-C gene. Preferably, the first amplification primer comprises a set of degenerate primers having between one and three degenerate sequence locations. More preferably, the first amplification primer comprises a set of two degenerate primers, the first degenerate primer of the set including the sequence GGCCCTGACCGAGACCTGGGC (SEQ ID #6), and the second degenerate primer of the set including the sequence GGCCCTGACCCAGACCTGGGC (SEQ ID #7), where the underlined nucleotides indicates the location of the GO C degeneracy. More preferably, the first amplification primer includes the sequence CATCCTGCTGCTCTCGGGAG (SEQ ID#30). Preferably, the second amplification primer includes the sequence CCACAGCTCCTAGGACAGCTAGGA (SEQ ID #8).

Preferably, the PCR method of the present invention is performed using the "hot start" process, e.g., Chou et al., Nucleic Acids Research 20: 1717-23 (1992). In the hot start process, a solid wax layer is formed over a subset of the PCR reactants. The remaining reactants are then added above the wax. In the first thermal cycle, rapid heating to the denaturation temperature melts the wax, whereupon thermal convection suffices to mix the upper and lower layers while the melted wax serves as a vapor barrier for the remainder of the amplification. The performance improvements realized by the hot start process follow from the reduction of primer oligomerization and mispriming side reactions that can occur during the initial stages of the PCR. Wax particles especially adapted for use with the hot start process are commercially available, e.g., from the Perkin-Elmer Corporation (PE p/n N808-0100; wherein "PE p/n" refers to a "Perkin-Elmer product number").

### 5. Sequence-Specific Detection

Once the desired HLA class I target sequence has been amplified, the resulting amplicon is then subjected to a sequence specific detection step. The sequence-specific detection methods for use with the present invention may include any method which is capable of distinguishing nucleic acid sequences differing by one or more nucleotides. Such methods include sequence-specific oligonucleotide probe hybridization (SSOP), restriction digestion with allele-specific restriction enzymes, DNA sequencing, and the like. The preferred sequence-specific detection method is DNA sequencing; the more preferred method being the Sanger-type dideoxy-mediated chain termination DNA sequencing method.

Two preferred labeling methods can be practiced with the present invention: (i) primer labeling methods, where the label is attached to the sequencing primer, e.g., Connell et al., Biotechniques 5(4): 342-348 (1987), or (ii) dideoxy terminator methods, where a label is attached to each of the dideoxy-A, -G, -C, or -T dideoxy terminators, e.g., Lee et al., Nucleic Acids Research 20(10): 2471-2483 (1992) and Prober et al., Science 238: 336-341 (1987). Suitable labels include any label which can be attached to a nucleotide, dideoxynucleotide, or polynucleotide in a chemically stable manner. Such labels include, fluorescent labels, chemiluminescent labels, spin labels, radioactive labels, and the like. The more preferred labeling method uses fluorescent labels which are attached to the sequencing primer.

Preferably, the sequencing method of the present invention sequences three exons of each the class I genes. For each of the HLA-A, -B, and -C genes, it is preferred that the exons to be sequenced include exon 2, exon 3, and exon 4. More preferably, each of the two DNA strands of each exon are sequenced, i.e., both the sense and antisense strands of the exon. By sequencing the exons in both directions, the effect of sequencing errors on the assignment of HLA type is minimized.

An important aspect of the present invention is that a single set of sequencing primers can be used to sequence the preferred exons 2, 3, and 4 of each of the HLA-A, -B, and -C class I genes, thereby greatly reducing the number of reagents and the complexity of the sequencing protocols required.

In a preferred embodiment, the sequencing primers are designed such that each primer is complementary to sequence located at an intron-exon boundary, thereby ensuring that the entire polymorphic portion of the exon of interest can be sequenced using a single sequencing primer. More preferably, the sequencing primers are between 10 nucleotides and 30 nucleotides in length.

Preferably, for obtaining the sequence of the antisense strand of exon 2, the sequencing primer is complementary to a region from - 20 nucleotides to + 20 nucleotides of the 5' intron-exon border of the sense strand of exon 2 (note in the above nomenclature, the "+" indicates a 5' to 3' direction along the polynucleotide strand and the "-" indicates a 3' to 5' direction along the polynucleotide strand). More preferably, the sequencing primer includes the sequence CACTCACCGGCCTCGCTCTGG (SEQ ID#12).

For obtaining the sequence of the sense strand of exon 2, the preferred sequencing primer is complementary to a region from +30 nucleotides to -20 nucleotides of the 5' intron-exon border of the antisense strand of exon 2. More preferably, the sequencing primer includes the sequences CTCGCCCCCAGGCTCCCAC (SEQ ID #9), AGGAGGGTCGGGCGGGTCTCAG (SEQ ID #31), or the degenerate sequences TCGGGCAGGTCTCAGCC (SEQ ID #25) and TCGGGCGGGTCTCAGCC (SEQ ID#26).

Preferably, for obtaining the sequence of the antisense strand of exon 3, the sequencing primer is complementary to a region from -30 nucleotides to +20 nucleotides of the 5' intron-exon border of the sense strand of exon 3. More preferably, the sequencing primer includes the sequences CCACTGCCCCTGGTACCCG (SEQ ID #13) or GAGGCGCCCCGTGGC (SEQ ID #29).

For obtaining the sequence of the sense strand of exon 3, the preferred sequencing primer is complementary to a region from +20 nucleotides to -20 nucleotides of the 5' intron-exon border of the antisense strand of exon 3. More preferably, the sequencing primer includes the sequences GCGGGGGCGGGTCCAGG (SEQ ID #10), GGGCTGACCACGGGGGCGGGGCCCAG (SEQ ID #32), or the degenerate sequences GGGCTCGGGGGACCGGG (SEQ ID#27) and GGGCTCGGGGGACTGGG (SEQ ID #28).

For obtaining the sequence of the antisense strand of exon 4, the preferred sequencing primer is complementary to a region from -30 nucleotides to +20 nucleotides of the 5' intron-exon border of the sense strand of exon 4. Preferably, the sequencing primer includes the sequence AGGGTGAGGGGCTTCGGCAGCC (SEQ ID#14)

For obtaining the sequence of the antisense strand of exon 4, the preferred sequencing primer is complementary to a region from +40 nucleotides to -10 nucleotides of the 5' intron-exon border of the antisense strand of exon 4. Preferably, the sequencing primer includes the sequence CTGACTCTTCCCATCAGACCC (SEQ ID#11).

The polymerase enzyme for use in the present invention can be any one of a number of possible known polymerase enzymes. Preferably, the polymerase is a thermostable polymerase, such as Taq DNA polymerase, a 94 kDa thermostable, recombinant DNA polymerase obtained by expression of a modified form of the Taq DNA polymerase gene in *E. coli,* e.g., Gelfand and White, PCR Protocols: A Guide to Methods and Applications, ed., Innis et al., Academic Press, CA, p129-141 (1991). The Taq DNA polymerase is preferred for PCR applications because of its optimal catalytic activity is in the same temperature range at which stringent annealing of primers to template DNA occurs, i.e., 55 °C to 75 °C. The Taq enzyme is commercially available from the Perkin-Elmer Corporation under the AmpliTaq™ trademark (PE p/n N801-0060).

A more preferred DNA polymerase for use in the instant invention is the TaqCS DNA polymerase. The TaqCS DNA polymerase is a single-point mutant of the Taq enzyme in which glycine number 46 has been replaced by an aspartic acid residue, i.e., a G46D mutant. Because the TaqCS enzyme coupled with cycle sequencing methods uses five to ten times less sequencing template as is required when using wildtype Taq enzyme, the use of the TaqCS enzyme obviates the need for any post-PCR purification prior to performing the sequencing reaction, resulting in a much simplified typing protocol. The TaqCS enzyme is available from the Perkin-Elmer Corporation under the AmpliTaqCS™ trademark. Another preferred variant of the AmpliTaq™ enzyme is the AmpliTaq™ DNA polymerase, FS, also available from Perkin-Elmer (p/n 402114)

### 6. Typing

Once the relevant DNA sequence information has been obtained, the sample is typed by comparing the experimentally determined DNA sequence (multiple sequences in the case of heterozygote samples) with well characterized sequences having known HLA types. Preferably the sequence comparison is performed using a computerized database.

### 7. Kits for Practicing the Preferred Embodiments of the Invention

The present invention lends itself readily to the preparation of kits containing the elements necessary to carry out the methods of the instant invention. As used herein, the term "kit" refers generally to a collection of containers containing the necessary elements to carry out the process of the invention in an arrangement both convenient to the user and which maximizes the chemical stability of the elements. Such a kit may comprise a carrier being compartmentalized to receive in close confinement therein one or more containers, such as tubes or vials, as well as printed instructions including a description of the most preferred protocols for carrying out the methods of the invention in a particular application, e.g., typing HLA-A, -B, and/or -C class I genes.

A first set of containers may contain the reagents for amplification of the desired target sequences. One container may contain an "upper" PCR amplification reagent, the reagent preferably including an aqueous solution of A, G, C, and T deoxynucleotides, and a polymerase enzyme, preferably a thermostable polymerase, e.g., TAQ polymerase. A second container may contain a "lower" PCR amplification reagent comprising an aqueous solution containing application-specific amplification primers, buffer, and other salts, e.g., magnesium. A third container may contain wax beads for use as a temporary liquid barrier between the upper and lower amplification reagents, e.g., the hot start process. Generally, the contents of the above kit components may be used as follows: (i) an aliquot of the lower amplification buffer may be added to a reaction container and a wax bead may be placed on top of the buffer; (ii) the contents of the reaction tube may then be heated to melt the wax, thereby forming a liquid barrier; (iii) the upper amplification buffer and the sample may then be added to the reaction container on top of the wax barrier; and finally, (iv) the above mixture may be subjected to thermal cycling, e.g., using a thermal cycler instrument.

A second set of containers may be included in the kits of the present invention, the second set comprising containers holding reagents for DNA sequencing. This set of containers may include four containers for each sequencing direction, i.e., 5' to 3' or 3' to 5' directions, of each locus to be sequenced, each container including application-specific sequencing primers, deoxy-and dideoxynucleotides, buffer, and other salts.

Clearly, other arrangements of containers and reagents may be used in the kits of the present invention. For example, some of the reagents of the kits may be supplied in a lyophilized state to enhance storage stability; the kits may include controls for calibration purposes; and, the kits may also include instructions describing preferred protocols for using the kits.

### 8. Examples

The invention will be further clarified by a consideration of the following examples, which are intended to be purely exemplary of the invention.

### EXAMPLE 1

### PCR Amplification of HLA-A, HLA-B, and HLA-C Class I Genes

PCR amplification was performed using a Perkin-Elmer 9600 thermal cycler. The amplification was performed with a wax-barrier hot start using Ampliwax™ wax pellets (PE p/n N808-0100). The amplification was performed on human genomic DNA that was purified from (i) peripheral blood cells, i.e., in the HLA-A and HLA-B examples, or (ii) from cultured cell lines, i.e., HLA-C where the BM21 cell line was used as the sample DNA source. The purification method used for all samples was the salting-out method, e.g., Miller et al., Nucleic Acids Research 16(3): 9-10 (1988). The average length of the purified genomic DNA was estimated to be at least 5 kilobases.

The amplification primers used for amplifying each of the HLA-A, -B, and -C genes are shown in Table I below.

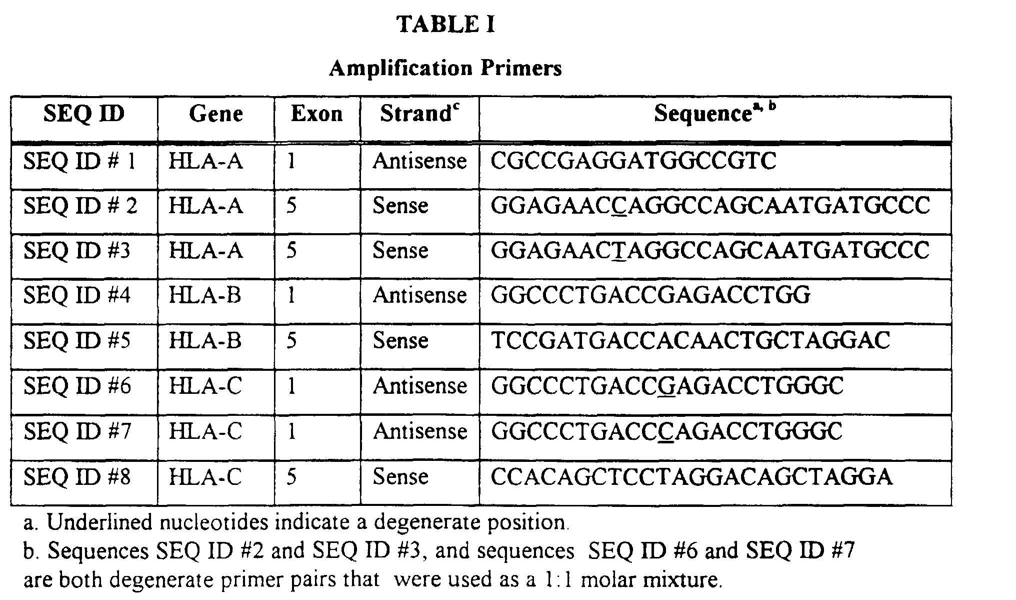

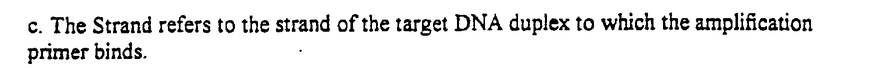

The PCR reaction for each of the HLA-A, -B, and -C genes was prepared as follows. First, a 5X concentrated PCR buffer was prepared having a final composition of 300 mM Tris[hydroxymethyl]aminomethane-HCl (Tris-HCl), 75 mM (NH₄)₂SO₄, and 7.5 mM MgCl₂, and having a pH of 9.0, such buffer being commercially available from Invitrogen Corporation, San Diego, CA (p/n Ki220-02-E). Next, 10 µl of the concentrated PCR buffer was mixed with 5 µl of a 10 mM deoxynucleotide triphosphate (dNTP) solution (2.5 mM each of dATP, dCTP, dGTP, and dTTP), 1 µl of a 10pmole/ µl solution of the sense-strand amplification primer(s), 1 µl of a 10 pmole/µl solution of the antisense-strand amplification primer(s), 2.5 µl of a 100 ng/ µl solution of the purified human genomic DNA, and, 20.5 µl of sterile double-distilled (dd) H₂0, resulting in a final volume of 40 µl.

Next, each of the above-prepared 40 µl reaction mixtures was added to a Microamp reaction tube (PE p/n N801-0533, N801-0534, N801-0540) containing one Ampliwax™ pellet (PE p/n N808-0100). The tube was then capped, briefly spun in a centrifuge at 3000 rpm to remove all droplets from the side of the tube, then heated to 65°C for 5 minutes to melt the Ampliwax™ pellet. The tube was then cooled to 4°C, thus forming a wax liquid barrier. A dilution of the AmpliTaq™ DNA polymerase enzyme (PE p/n N801-0060) was then made to a final concentrations of 0.1 U/ µl in sterile ddH₂0. This enzyme solution was then layered on top of the wax liquid barrier.

The reaction tubes were then placed in a Perkin-Elmer 9600 thermal cycler, denatured at 98 °C for 20 s, then the following thermal cycle was run: 98 °C for 5 s followed by 68 °C for 2 min, where the cycle was repeated eight times; and 96 °C for 5 s followed by 70 °C for 2 min, where the cycle was repeated 32 times.

Following thermocycling, 8 µl of each of the reactions was analyzed by agarose gel electrophoresis to ensure proper PCR amplification. A 0.7% agarose gel was used containing ethidium bromide at 0.8 µg/ml and TBE buffer (89mM Tris-HCl, 89mM Boric Acid, 2mM Na₂EDTA, pH8.3) as both the gel and running buffer. The gel was electrophoresed at 7 V/cm for 2 hrs and visualized using a UV transilluminator. A band at about 2.0 kb for the HLA-A, and -B, or C products was seen indicating successful amplification of each of the specific genes (sizes based on internal size standards).

### EXAMPLE 2

### DNA Sequencing of Exons 2, 3, and 4 of the HLA-A, HLA-B, and HLA-C Class I Genes Using the Amplification Products from Example 1

Sequencing was performed with no purification of the PCR products using the TaqCS polymerase enzyme.

The sequencing primers shown below in Table II were used to sequence exons 2, 3 and 4 of HLA-A, B and C genes.

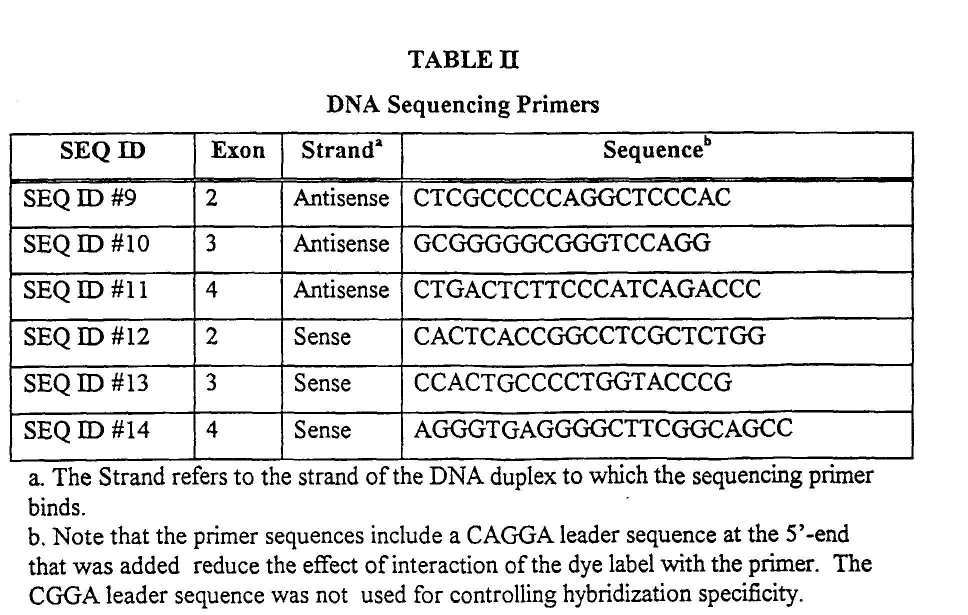

Each of the above primers is labeled at the 5'-end with one of the four fluorescent dyes 5-carboxy-fluorescein (FAM), 2',7'-dimethoxy-4', 5'-dichloro-6-carboxy-fluorescene (JOE), N,N,N',N'-tetramethyl-6-carboxyrhodamine (TAMRA), and 6-carboxy-X-rhodamine (ROX), e.g., U.S. Pat. No. 4,855,225.

The sequencing reaction mixes were prepared as shown in Tables III and IV, each reaction being prepared in a Microamp tube (PE p/n N801-0533, N801-0534, N801-0540) while placed in crushed ice. Note that four reaction mixes were prepared for each sequencing primer providing one reaction mix for each A, G, C, or T termination reaction.

**TABLE III**

| **Sequencing reaction Mixes for Sequencing Primers SEQ ID #12, SEQ ID #13, SEQ ID #14, and SEQ ID #15** | | | | |
|---|---|---|---|---|
| | A | C | G | T |
| 5X Seq. Buffer^{a} | 1µl | 1µl | 2µl | 2µl |
| A d/dd^{b} | 1µl | - | - | - |
| C d/dd^{c} | - | 1µl | - | - |
| G d/dd^{d} | - | - | 2µl | - |
| T d/dd^{e} | - | - | - | 2µl |
| A Dye Primer^{f} | 1µl | - | - | - |
| C Dye Primer^{f} | - | 1µl | - | - |
| G Dye Primer^{f} | - | - | 2µl | - |
| T Dye Primer^{f} | - | - | - | 2µl |
| Taq dilution^{g} | 1µl | 1µl | 2µl | 2µl |
| Diluted Template^{h} | 1µl | 1µl | 2µl | 2µl |

| | | | | |
|---|---|---|---|---|
| a. The composition of the 5X Seq. Buffer was 400 mM Tris-HCl, 10 mM MgCl₂, pH 9.0. | | | | |
| b. The composition of the A d/dd mix was 900 µM ddATP, 93.75 µM dATP, 250 µM dCTP, 375 µM7-deaza-dGTP, and 250 µM dTTP. | | | | |
| c. The composition of the C d/dd mix was 450 µM ddCTP, 250 µM dATP, 93.75 µM dCTP, 375 µM7-deaza-dGTP, and 250 µM dTTP. | | | | |
| d. The composition of the G d/dd mix was 90 µM ddGTP, 250 µM dATP, 250 µM dCTP, 180 µM7-deaza-dGTP, and 250 µM dTTP. | | | | |
| e. The composition of the T d/dd mix was 1250 µM ddGTP, 250 µM dATP, 250 µM dCTP, 180 µM7-deaza-dGTP, and 250 µM dTTP. | | | | |
| f. Each primer solution was made at a concentration of 0.4 pmoles/µl. | | | | |
| g. The TaqCS polymerase was diluted by mixing 1 µl of a TaqCS stock solution with 5 µl of the 5X concentrated sequencing buffer from (a). The TaqCS stock was at a concentration of 5 Units/µl where one unit is defined as in Lawyer et al., J. Biol. Chem. 264:6427-6437 (1989) herein incorporated by reference. | | | | |
| h. The template was diluted by mixing 1 µl of the PCR product from Example 1 with 5 µl water. | | | | |

**TABLE IV**

| **Sequencing Reaction Mixes for Sequencing Primers SEQ ID #9 and SEQ ID #10** | | | | |
|---|---|---|---|---|
| | A | C | G | T |
| 5X Seq. Buffer^{a} | 1µl | 1µl | 2µl | 2µl |
| A d/dd^{b} | 1µl | - | - | - |
| C d/dd^{c} | - | 1µl | - | - |
| G d/dd^{c} | - | - | 2µl | - |
| T d/dd^{e} | - | - | - | 2µl |
| A Dye Primer^{f} | 2µl | - | - | - |
| C Dye Primer^{f} | - | 2µl | - | - |
| G Dye Primer^{f} | - | - | 4µl | - |
| T Dye Primer^{f} | - | - | - | 4µl |
| Taq dilution^{g} | 1µl | 1µl | 2µl | 2µl |
| Diluted Template^{h} | 1µl | 1µl | 2µl | 2µl |
| a-f. These notes have the same meaning as those in Table III above. | | | | |

Each of the sequencing reaction mixes were denatured at 98 °C for 5 s then thermocycled in a Perkin-Elmer 9600 thermocycler using the following program: 96 °C for 5 s followed by 55 °C for 40 s, followed by 68 °C for 1 min, where the cycle was repeated 15 times; and 96 °C for 5 s followed by 68 °C for 1 min, where the cycle was repeated 15 times.

After completion of the sequencing reactions, each of the A, G, C, and T reactions for each gene were pooled and added to 200 µl of 95% Ethanol on ice. After allowing the DNA to precipitate, the tubes were centrifuged at 17, 000 rpm for 20 min, whereupon the ethanol was decanted by vacuum aspiration. Next, 200 µl of 70% ethanol was added to the dry DNA pellet, vortexed vigorously, then centrifuged at 17,000 rpm for 15 min, then dried in a vacuum centrifuge set to medium heat for 5 minutes. The precipitated samples were then resuspended and loaded on a standard DNA sequencing gel, and run on an automated fluorescent DNA sequencer as described in the Applied Biosystems 373A manual (373A DNA Sequencing System Users Manual, Sections 2, 3, and 4, p/n 901156, Software version 1.10, Document Rev. C, January 1992, ABI).

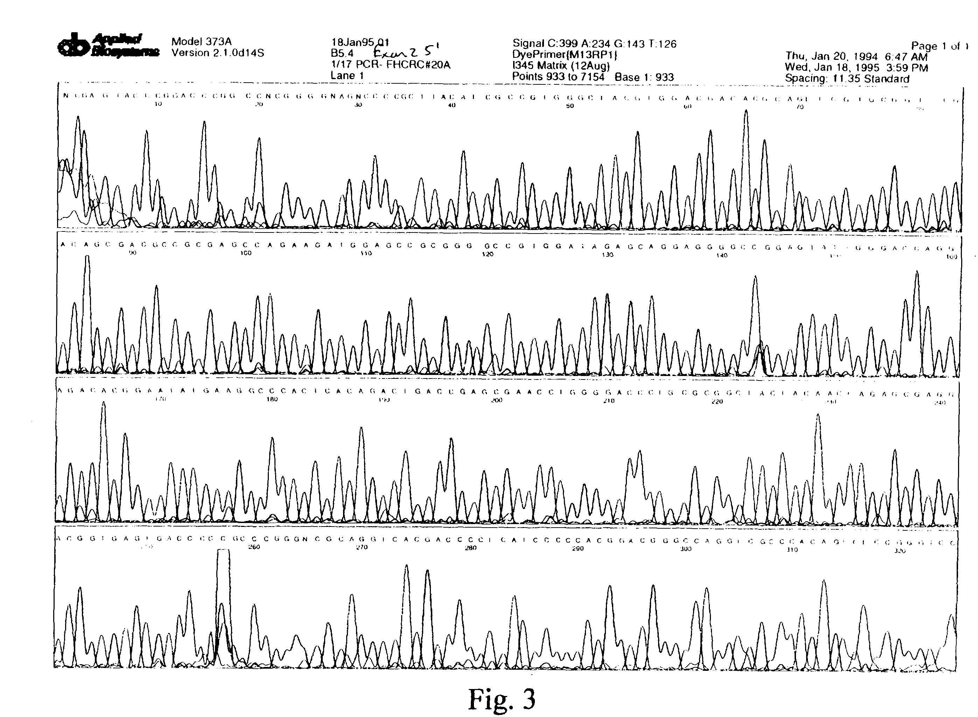

The following Figs. 3-21 show examples of HLA class I typing data collected using the protocols of Examples 1 and 2.

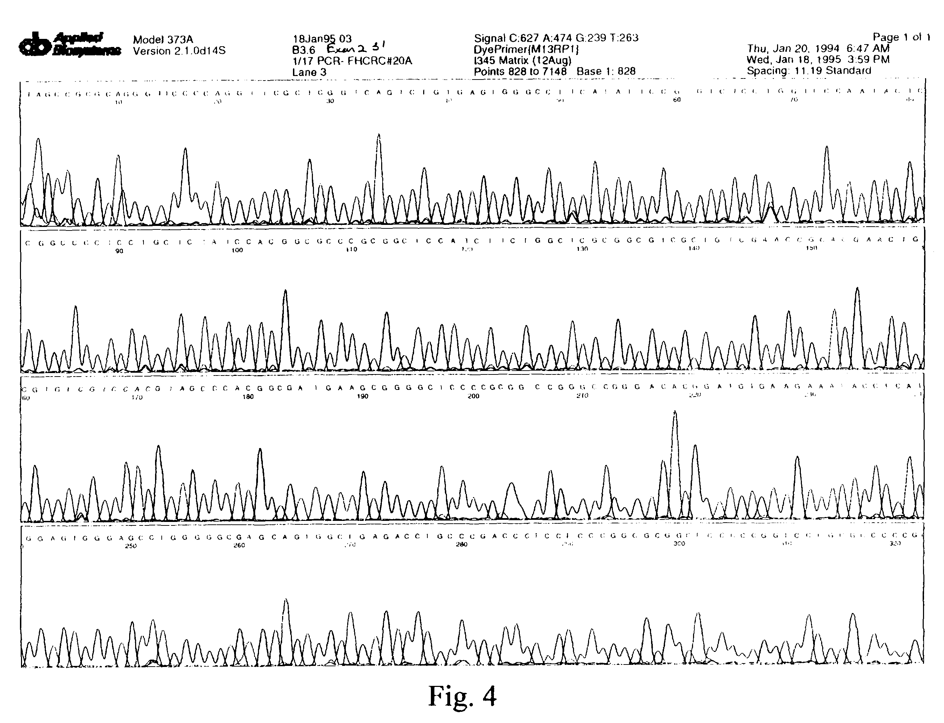

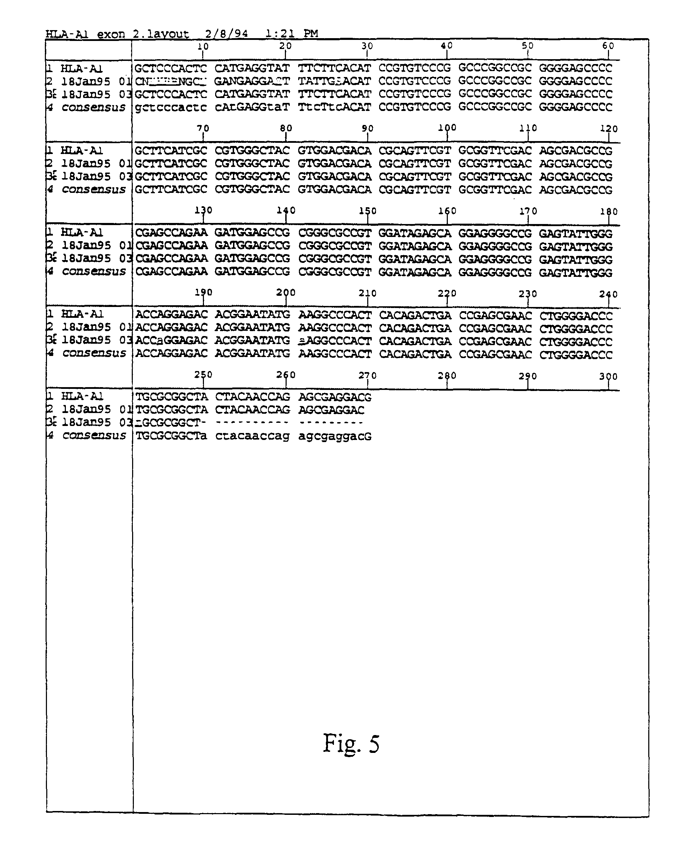

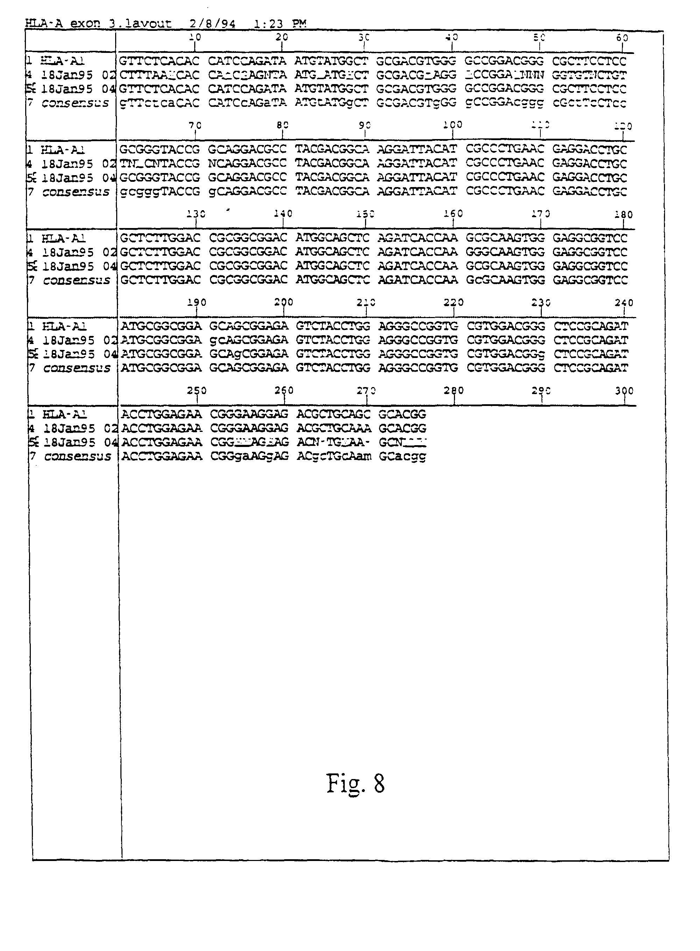

Figs. 3-8 show typing data from the HLA-A class I gene. Figs. 3 and 4 show the raw sequence of the sense strand and the antisense strand of exon 2 of the HLA-A gene using the sequencing primers SEQ ID #9 and SEQ ID #12, respectively. Fig. 5 shows a sequence alignment of the data from Figs. 3 and 4 where: line 1 is the reference sequence of the putative allele, HLA-A0101 (Genbank Accession No. M24043); line 2 is the experimentally determined sequence of the sense strand obtained from Fig. 3; line 3 is the experimentally determined sequence of the antisense strand obtained from Fig. 4; and, line 4 is a consensus sequence derived from the sequences in lines 1, 2, and 3 (note that the line numbers 1, 2, 3, and 4 refer to the numbers in the leftmost column of each of the sequence panels in Fig. 5 and each of the following alignment figures, i.e., Figs 8, 11, 16, 19, and 21).

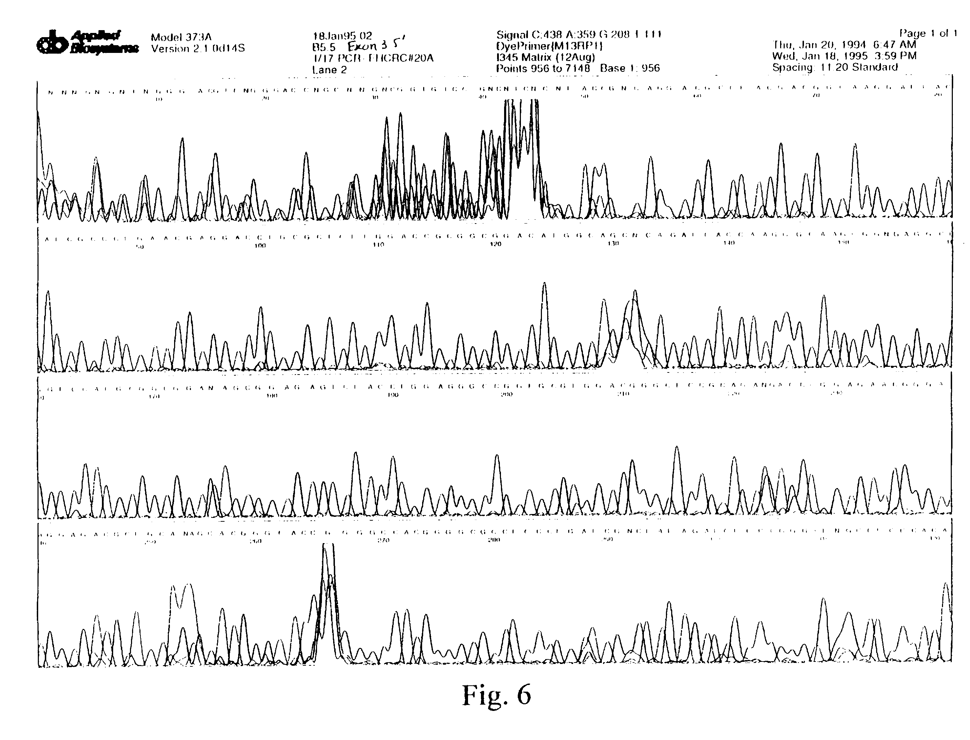

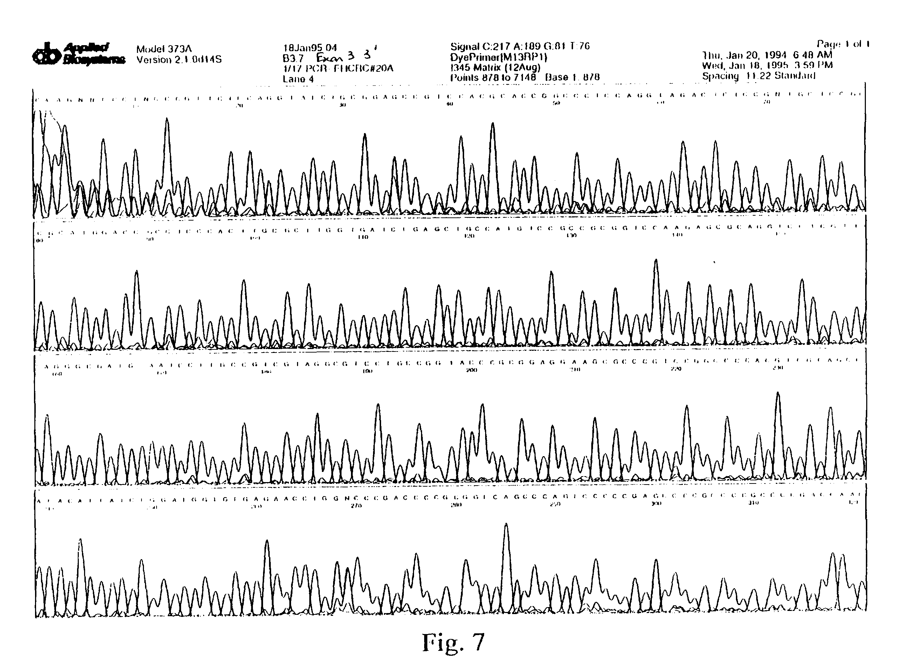

Figs. 6 and 7 show the raw sequence of the sense strand and the antisense strand of exon 3 of the HLA-A gene using the sequencing primers SEQ ID #10 and SEQ ID #14, respectively. Fig. 8 shows a sequence alignment of the data from Figs. 6 and 7 where: line 1 is the reference sequence of the putative allele, HLA-A0101 (or HLA-A1) (Genbank Accession No. M24043); line 4 is the experimentally determined sequence of the sense strand obtained from Fig. 6; line 5 is the experimentally determined sequence of the antisense strand obtained from Fig. 7; and line 7 is a consensus sequence derived from the sequences in lines 1, 4, and 5.

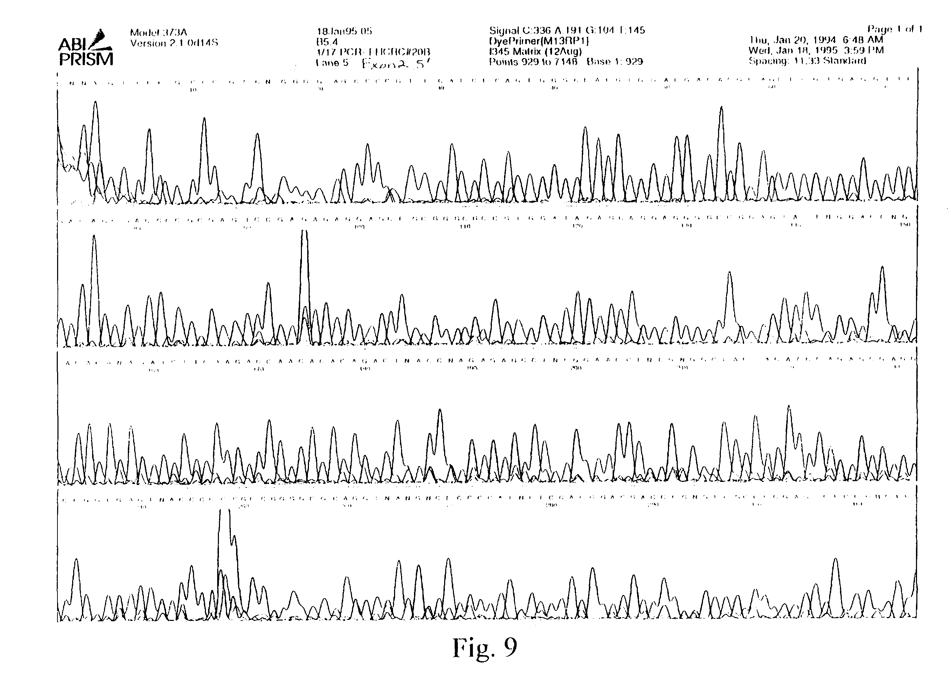

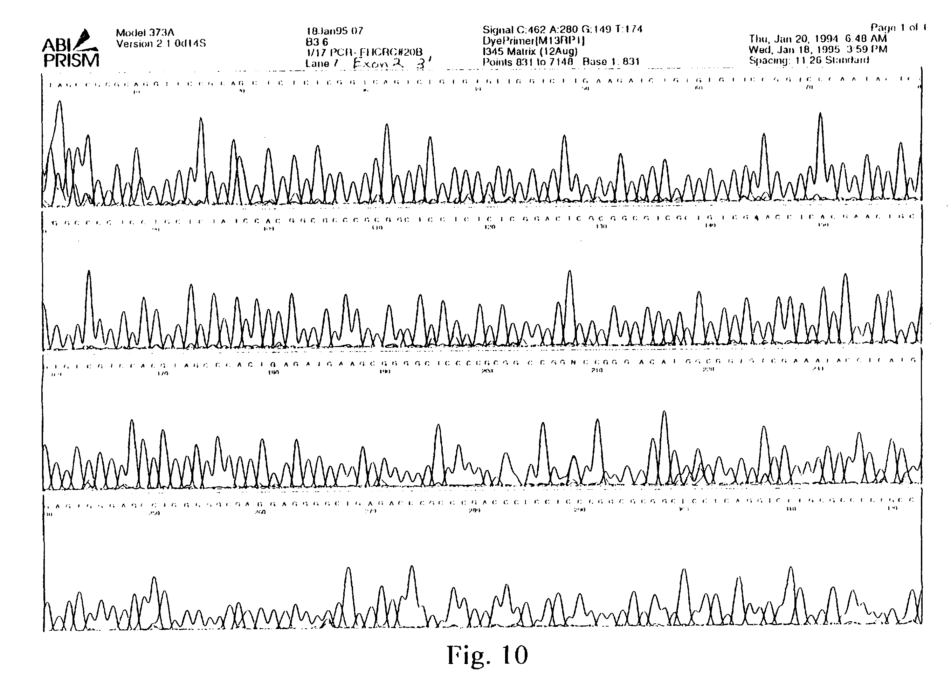

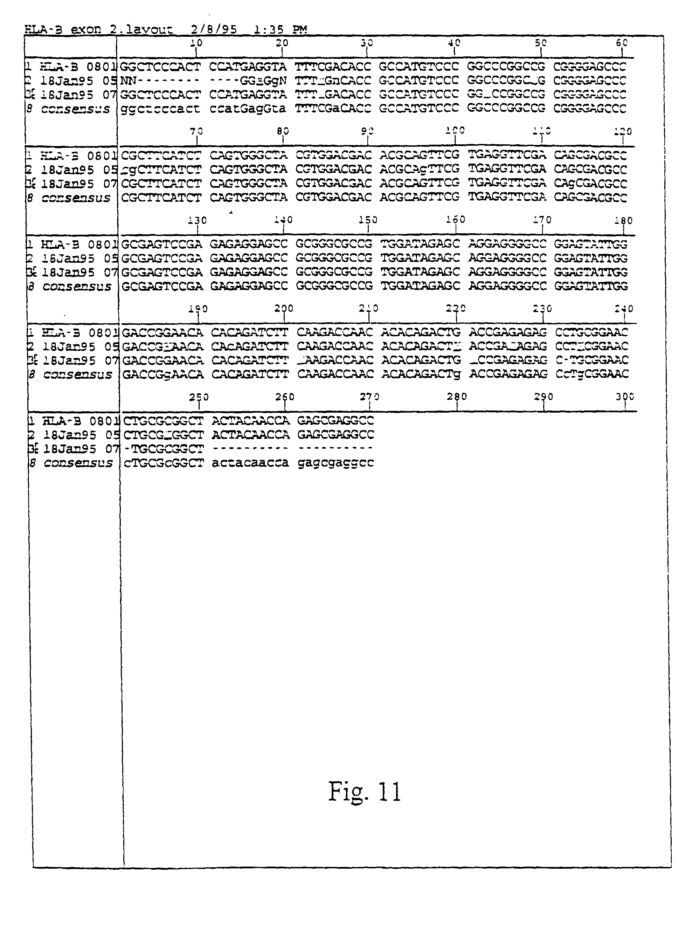

Figs. 9-16 show typing data from the HLA-B class I gene. Figs. 9 and 10 show the raw sequence of the sense and antisense strands of exon 2 of the HLA-B gene using the sequencing primers SEQ ID #9 and SEQ ID #13, respectively. Fig. 11 shows a sequence alignment of the data from Figs. 9 and 10 where: line 1 is the reference sequence of the putative allele, HLA-B0801 (Genbank Accession No. M24036); line 2 is the experimentally determined sequence of the sense strand obtained from Fig. 9; line 3 is the experimentally determined sequence of the antisense strand obtained from Fig. 10; and, line 8 is a consensus sequence derived from the sequences in lines 1, 2 and 3.

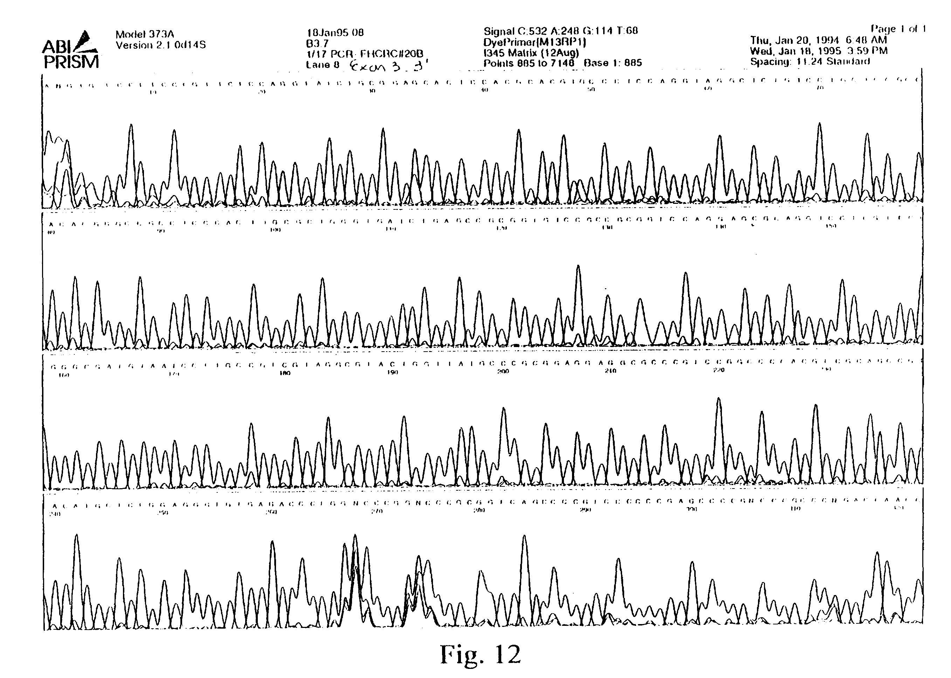

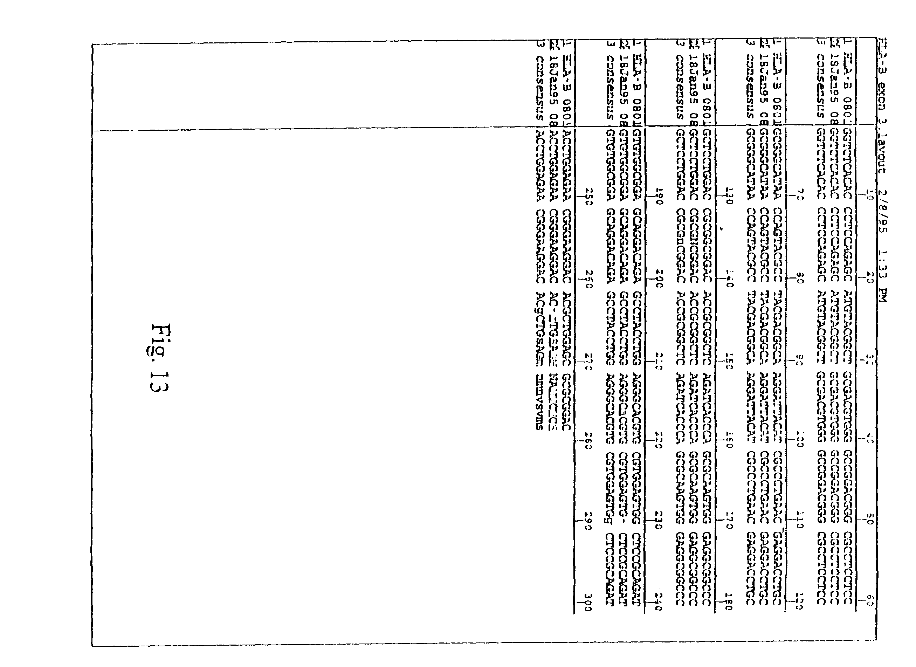

Fig. 12 shows the raw sequence of the antisense strand of exon 3 of the HLA-B gene using the sequencing primer SEQ ID #14. Fig. 13 shows a sequence alignment of the data from Fig . 12 where: line 1 is the reference sequence of the putative allele, HLA-B0801 (Genbank Accession No. M24036); line 2 is the experimentally determined sequence of the antisense strand obtained from Fig. 12; and line 3 is a consensus sequence derived from the sequences in lines 1 and 2.

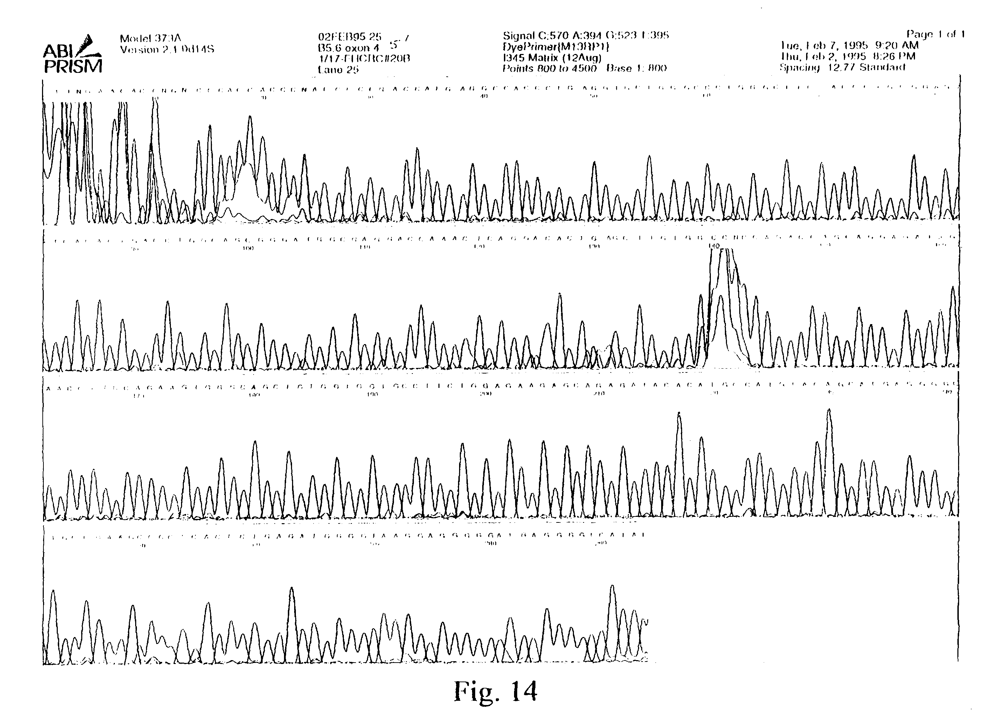

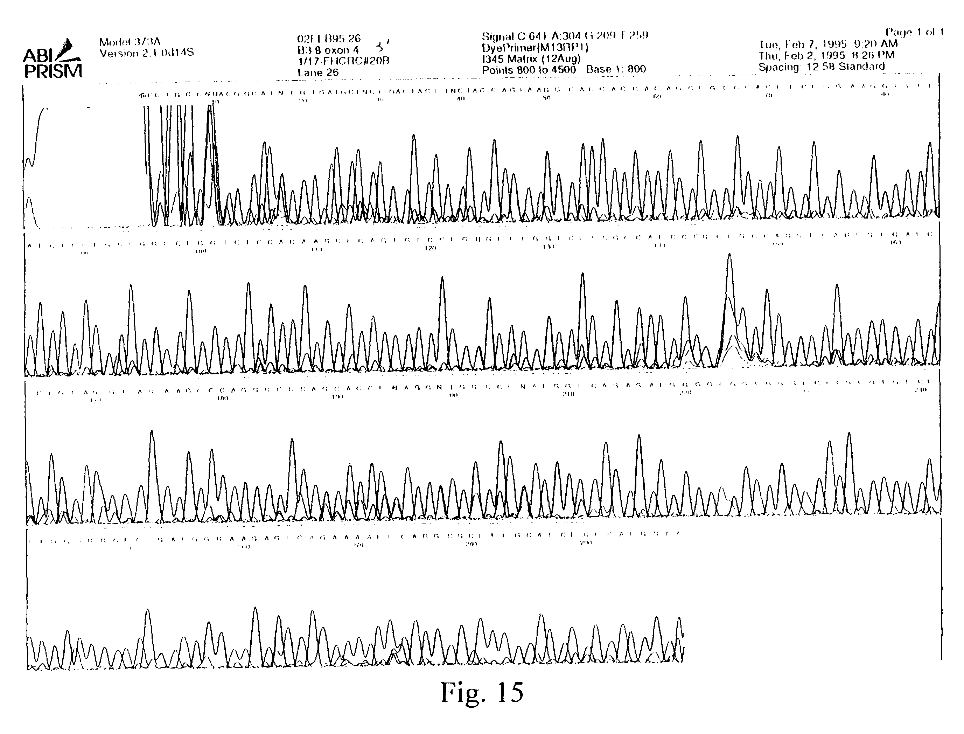

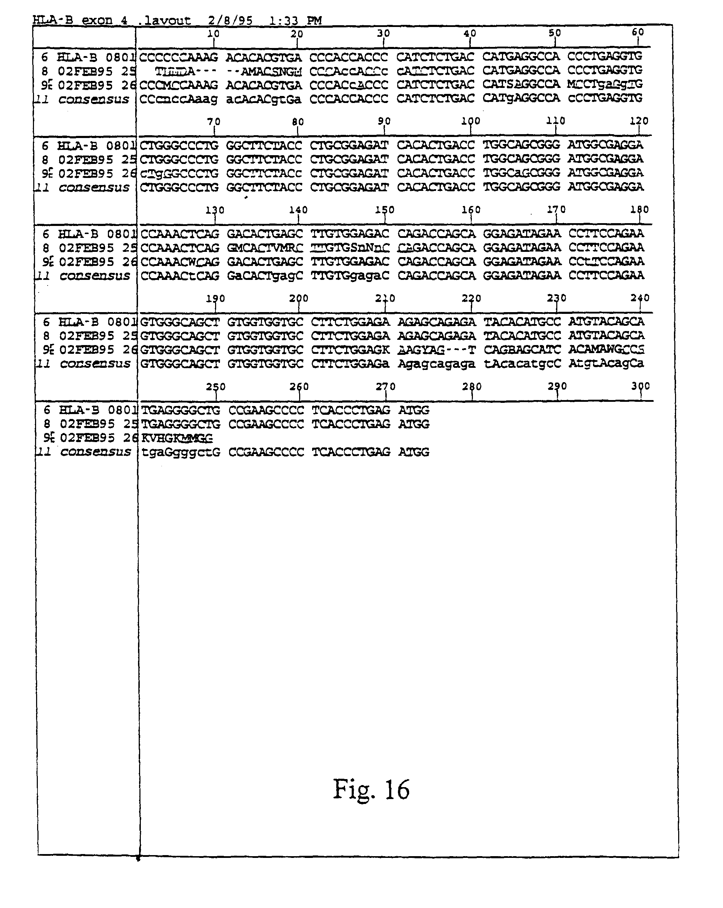

Figs. 14 and 15 show the raw sequence of the sense and antisense strands of exon 4 of the HLA-B gene using the sequencing primers SEQ ID #12 and SEQ ID #15, respectively. Fig. 16 shows a sequence alignment of the data from Figs. 14 and 15 where: line 6 is the reference sequence of the putative allele, HLA-B0801 (Genbank Accession No. M24036); line 8 is the experimentally determined sequence of the sense strand obtained from Fig. 14; line 9 is the experimentally determined sequence of the antisense strand obtained from Fig. 15; and, line 11 is a consensus sequence derived from the sequences in lines 6, 8 and 9.

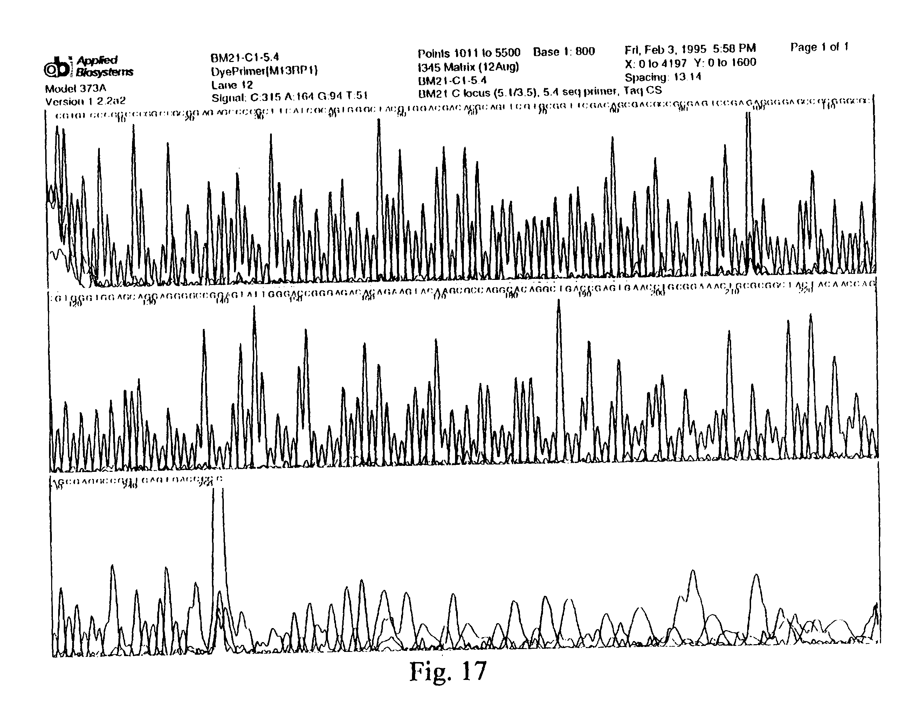

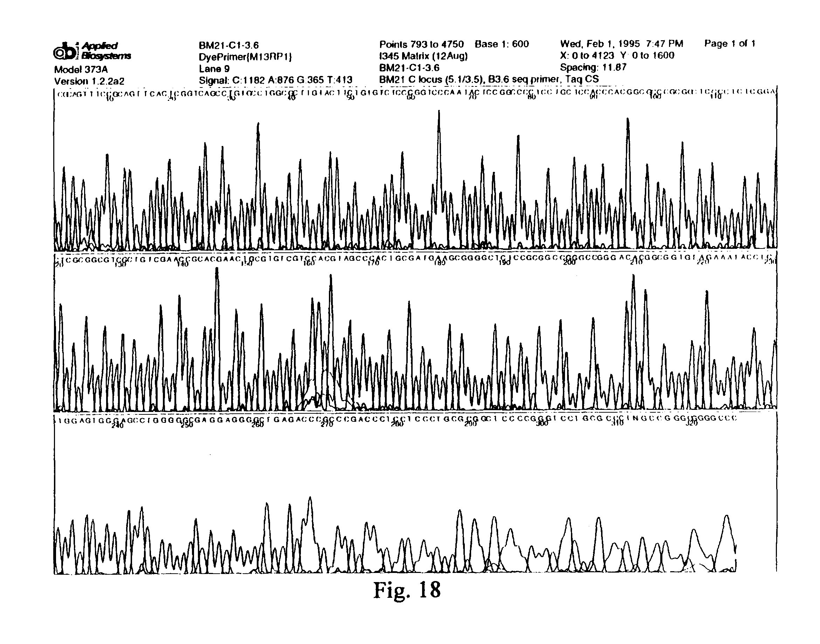

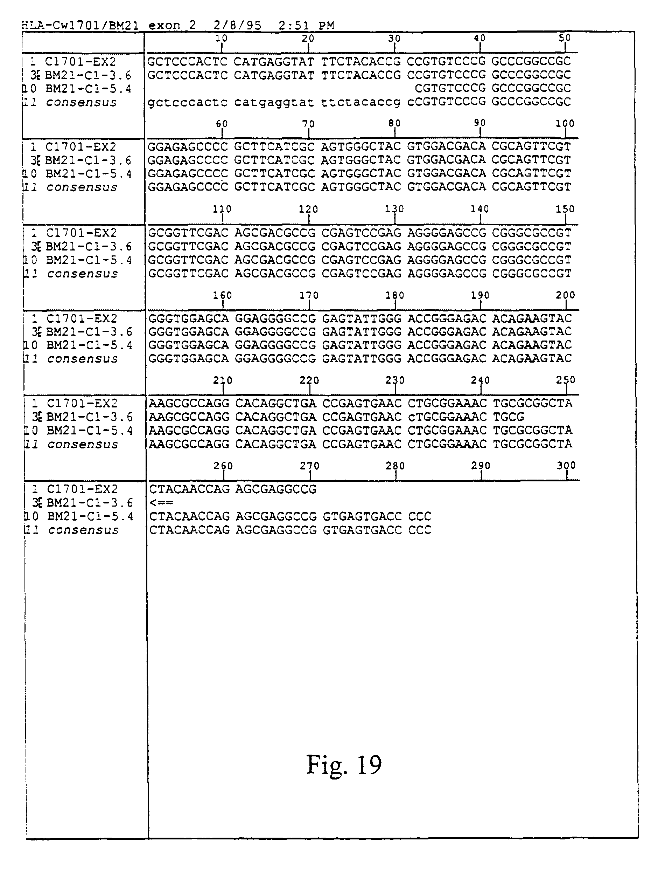

Figs. 17 and 18 show the raw sequence of the sense and antisense strand of exon 2 of the HLA-C gene using the sequencing primers SEQ ID #9 and SEQ ID #13, respectively. Fig. 19 shows a sequence alignment of the data from Figs. 17 and 18 where: line 1 is the reference sequence of the putative allele, HLA-CW1701 (Genbank Accession No. U06835); line 10 is the experimentally determined sequence of the sense strand obtained from Fig. 17; line 3 is the experimentally determined sequence of the antisense strand obtained from Fig. 18; and, line 11 is a consensus sequence derived from the sequences in lines 1, 3 and 10.

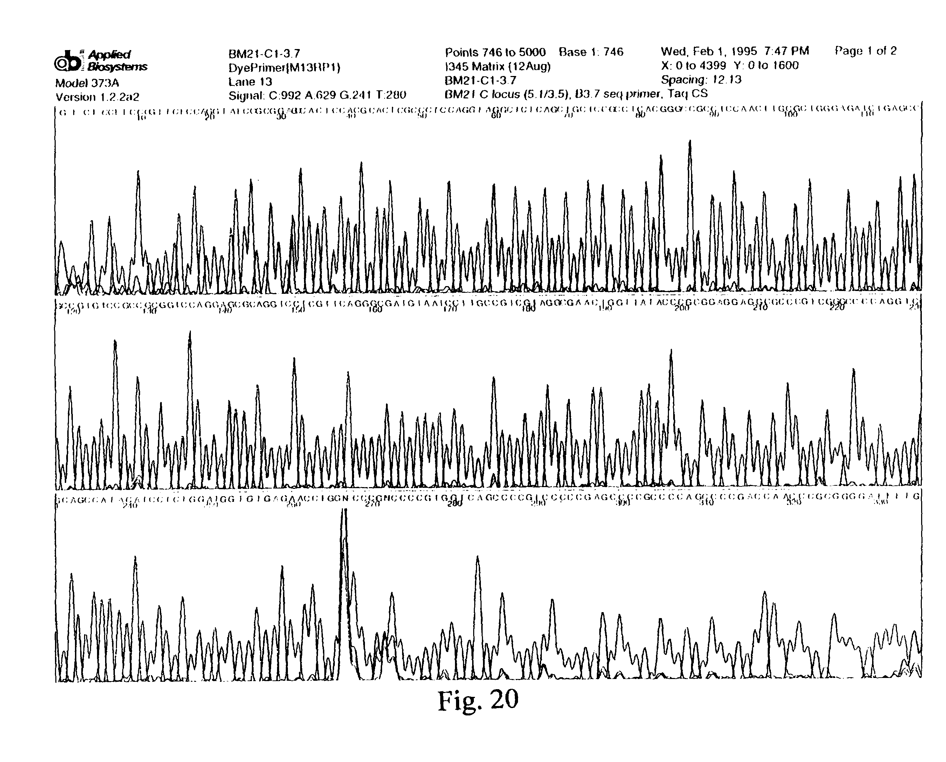

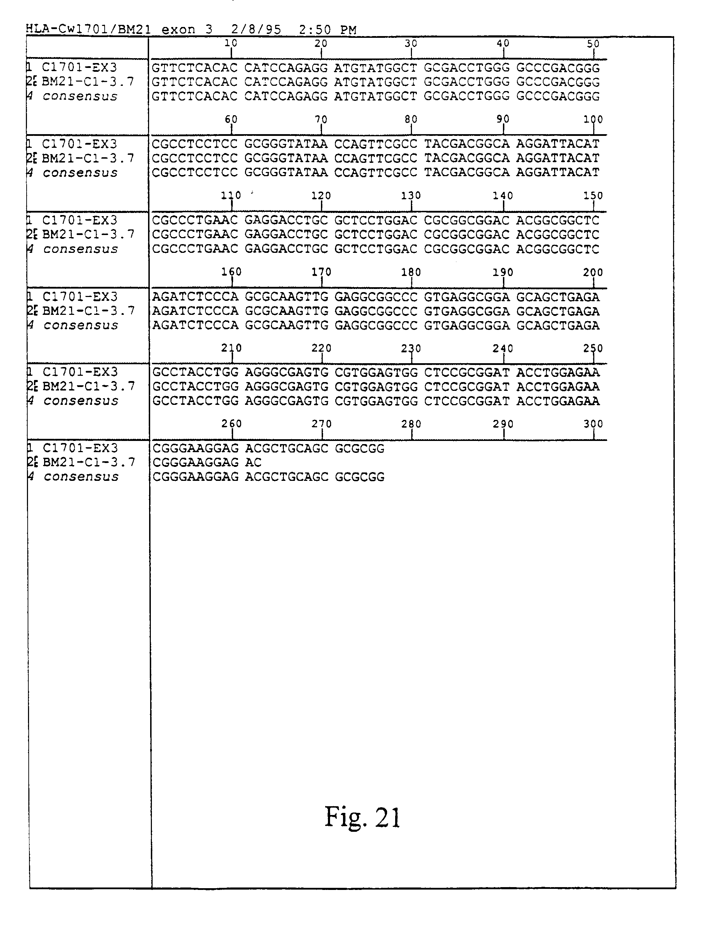

Fig. 20 shows the raw sequence of the antisense strand of exon 3 of the HLA-C gene using the sequencing primer SEQ ID #14. Fig. 21 shows a sequence alignment of the data from Fig. 20 where: line 1 is the reference sequence of the putative allele, HLA-C1701 (Genbank Accession No. U06835); line 2 is the experimentally determined sequence of the antisense strand obtained from Fig. 20; and line 4 is a consensus sequence derived from the sequences in lines 1 and 2.

### EXAMPLE 3

### PCR Amplification of HLA-A and HLA-B Class I Genes

PCR amplification was performed using a Perkin-Elmer 9600 thermal cycler. The amplification was performed with a wax-barrier hot start using Ampliwax™ wax pellets (PE p/n N808-0100). The amplification was performed on human genomic DNA that was purified from peripheral blood cells using the salting-out method, e.g., Miller et al., *supra.* The average length of the purified genomic DNA was estimated to be at least 5 kilobases. The amplification primers used for amplifying each of the HLA-A and HLA-B genes are shown in Table V below.

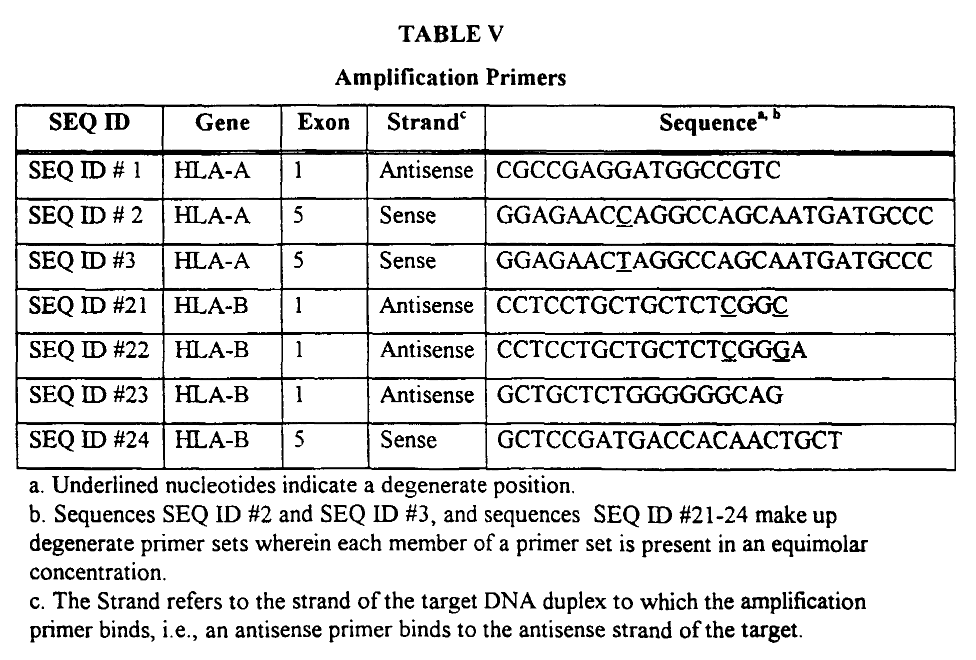

The PCR reaction for the HLA-A gene was prepared as follows. Four µl of the 5X concentrated PCR buffer from Example 1 was mixed with 5 µl of a 10 mM deoxynucleotide triphosphate (dNTP) solution (2.5 mM each of dATP, dCTP, dGTP, and dTTP), 1 µl of a 10 pMole/ µl solution of the SEQ ID #2 and SEQ ID #3 amplification primer(s), 2.5 µl of a 100 ng/ µl solution of the purified human genomic DNA, and, a volume of sterile double-distilled (dd) H₂0 sufficient to result in a final volume of 30 µl.

Next, the above-prepared 30 µl reaction mixture was added to a Microamp reaction tube (PE p/n N801-0533, N801-0534, N801-0540) containing one Ampliwax™ pellet (PE p/n N808-0100). The tube was capped, briefly spun in a centrifuge at 3000 rpm to remove all droplets from the side of the tube, then heated to 65°C for 5 minutes to melt the Ampliwax™ pellet. The tube was then cooled to 4°C, thus forming a wax liquid barrier.

Above the wax barrier was added a solution containing 6µl of the 5X concentrated PCR buffer, 1 µl of a 10 pMole/ µl solution of the SEQ ID #1 amplification primer, 1U of AmpliTaq™ DNA polymerase, and a volume of sterile double-distilled (dd) H₂0 sufficient to result in a final volume of 30 µl.

The PCR reaction for the HLA-B gene was prepared as follows. Four µl of the 5X concentrated PCR buffer from Example 1 was mixed with 5 µl of a 10 mM deoxynucleotide triphosphate (dNTP) solution (2.5 mM each of dATP, dCTP, dGTP, and dTTP), 1 µl of a 10 pmole/ µl solution of the SEQ ID #24 amplification primer, 2.5 µl of a 100 ng/ µl solution of the purified human genomic DNA, and, a volume of sterile double-distilled (dd) H₂0 sufficient to result in a final volume of 20 µl,

Next, the above-prepared 20 µl reaction mixture was added to a Microamp reaction tube containing one Ampliwax™ pellet . The tube was capped, briefly spun in a centrifuge at 3000 rpm to remove all droplets from the side of the tube, then heated to 65°C for 5 minutes to melt the Ampliwax™ pellet. The tube was then cooled to 4°C, thus forming a wax liquid barrier.

Above the wax barrier was added a solution containing 6µl of the 5X concentrated PCR buffer, 1 µl of a 15 pMole/ µl solution of the SEQ ID #21, SEQ ID #22, and SEQ ID #23 amplification primers, 1U of AmpliTaq™ DNA polymerase, and a volume of sterile double-distilled (dd) H₂0 sufficient to result in a final volume of 30 µl.

The above-prepared reaction tubes were placed in a Perkin-Elmer 9600 thermal cycler, denatured at 98 °C for 20 s, then subjected to the following thermal cycle program: (i) 98 °C for 5 s, 65 °C for 30 s, 72 °C for 2 min, where the cycle was repeated eight times; followed by (ii) 96 °C for 5 s, 60 °C for 30 s, 72 °C for 2 min, where the cycle was repeated 32 times.

Following thermocycling, 8 µl of each of the reactions was analyzed by agarose gel electrophoresis to ensure proper PCR amplification. A 0.7% agarose gel was used containing ethidium bromide at 0.8 µg/ml and TBE buffer (89mM Tris-HCl, 89mM Boric Acid, 2mM Na₂EDTA, pH8.3) as both the gel and running buffer. The gel was electrophoresed at 7 V/cm for 2 hrs and visualized using a UV transilluminator. A band at about 2.0 kb for the HLA-A, or -B, products was seen indicating successful amplification of each of the specific genes (sizes based on internal size standards).

### EXAMPLE 4

### DNA Sequencing of Exons 2, 3, and 4 of the HLA-A and HLA-B Class I Genes Using the Amplification Products from Example 3

Sequencing was performed with no purification of the PCR products using the AmpliTaq® DNA polymerase FS enzyme from Perkin-Elmer (p/n 402114). The sequencing protocol used was that suggested by Perkin-Elmer with some minor modifications, e.g., ABI Prism Dye Primer Cycle Sequencing Core Kit Protocol, Revision A, July 1995, such reference incorporated herein by reference.

The sequencing primers shown below in Table VI were used to sequence exons 2 and 3 of the HLA-A and HLA-B genes.

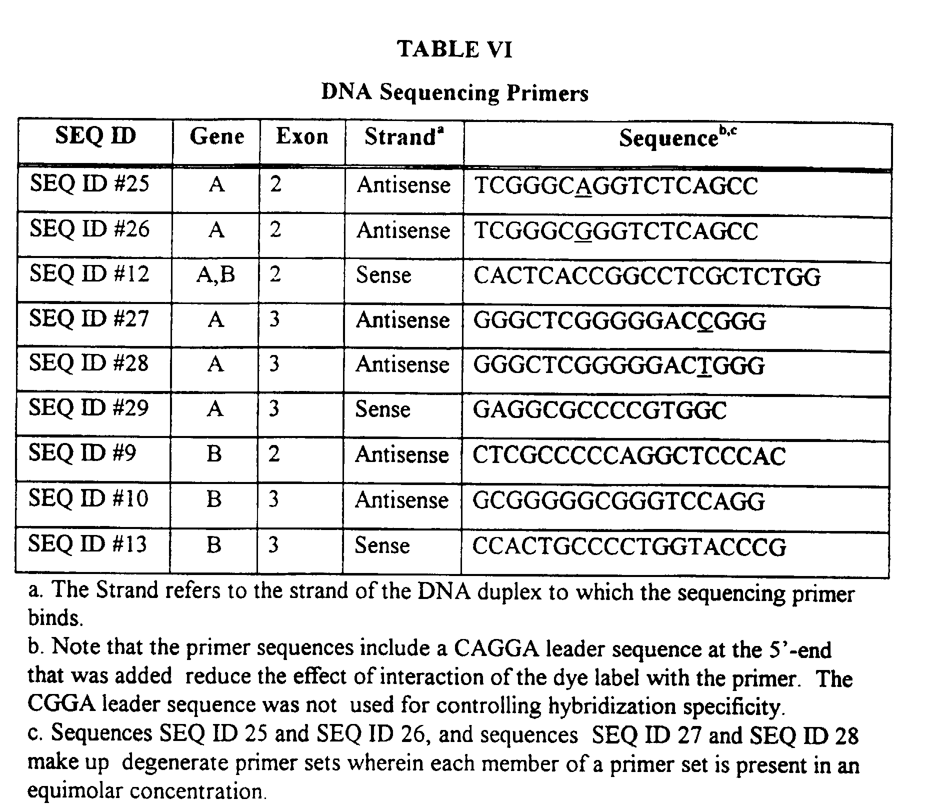

Each of the primers in Table VI is labeled at the 5'-end with one of the four fluorescent dyes 5-carboxy-fluorescein (FAM), 2',7'-dimethoxy-4', 5'-dichloro-6-carboxy-fluorescene (JOE), N,N,N',N'-tetramethyl-6-carboxyrhodamine (TAMRA), and 6-carboxy-X-rhodamine (ROX), e.g., U.S. Pat. No. 4,855,225.

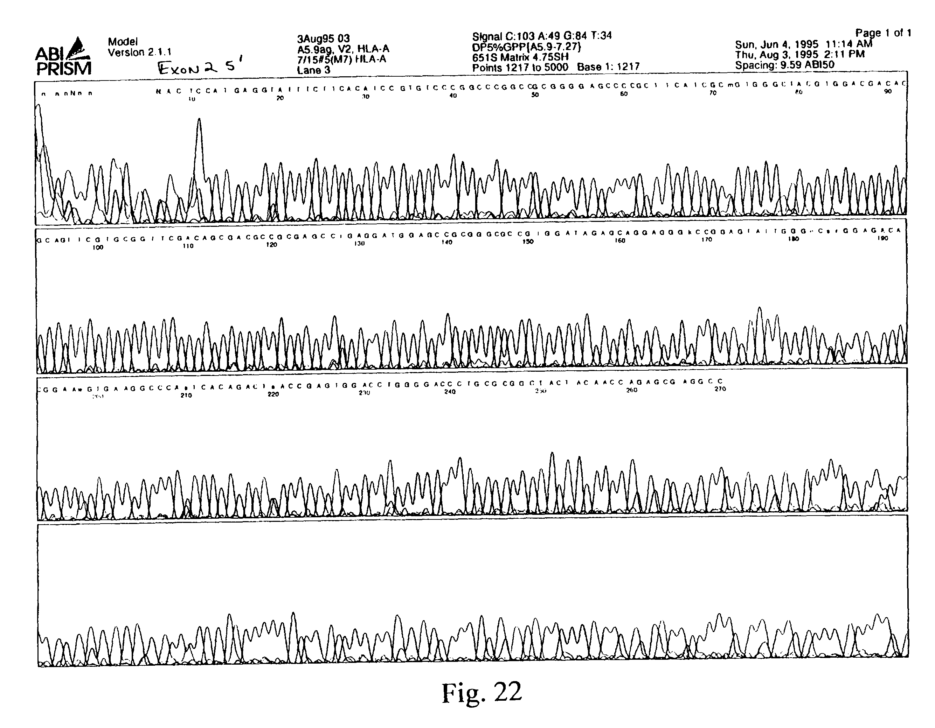

The following Figs. 22-33 show examples of HLA class I typing data collected using the protocols of Examples 3 and 4.

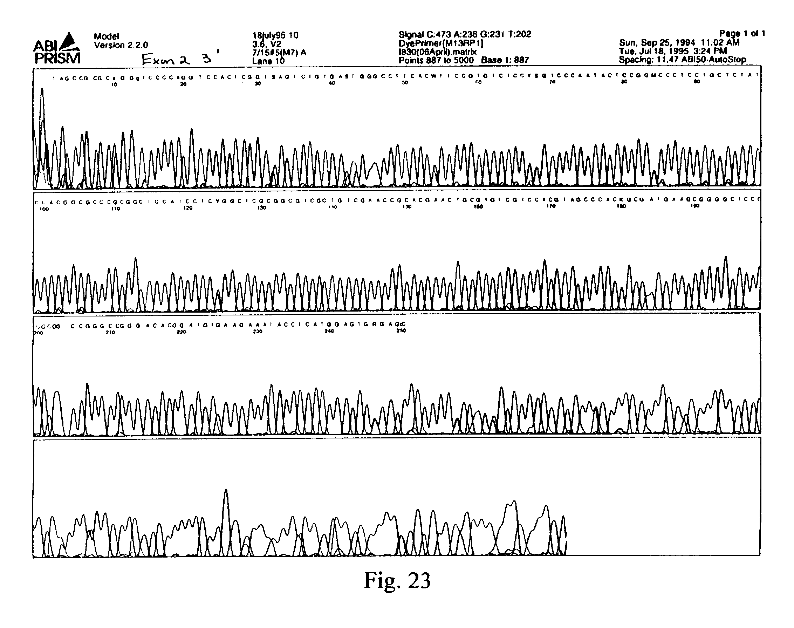

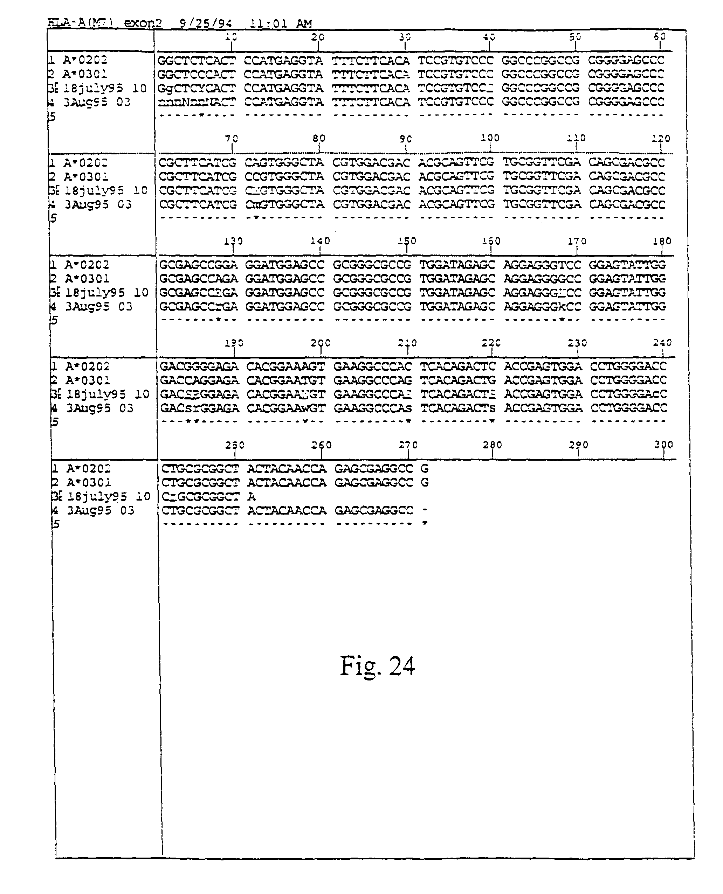

Figs. 22-27 show typing data from the HLA-A class I gene. Figs. 22 and 23 show the raw sequence of the sense strand and the antisense strand of exon 2 of the HLA-A gene, respectively. Sequencing primers SEQ ID #25 and SEQ ID #26 were used to obtain the sequence of the sense strand shown in Fig. 22, and sequencing primer SEQ ID #12 was used to obtain the sequence of the antisense strand shown in Fig 23. Fig. 24 shows a sequence alignment of the data from Figs. 22 and 23 where: lines 1 and 2 are the reference sequence of the putative diploid alleles, HLA-A0202 and HLA-A0301, respectively; line 3 is the experimentally determined sequence of the sense strand obtained from Fig. 22; and line 4 is the experimentally determined sequence of the antisense strand obtained from Fig. 23 (note that the line numbers 1, 2, 3, and 4 refer to the numbers in the leftmost column of each of the sequence panels in Fig. 24).

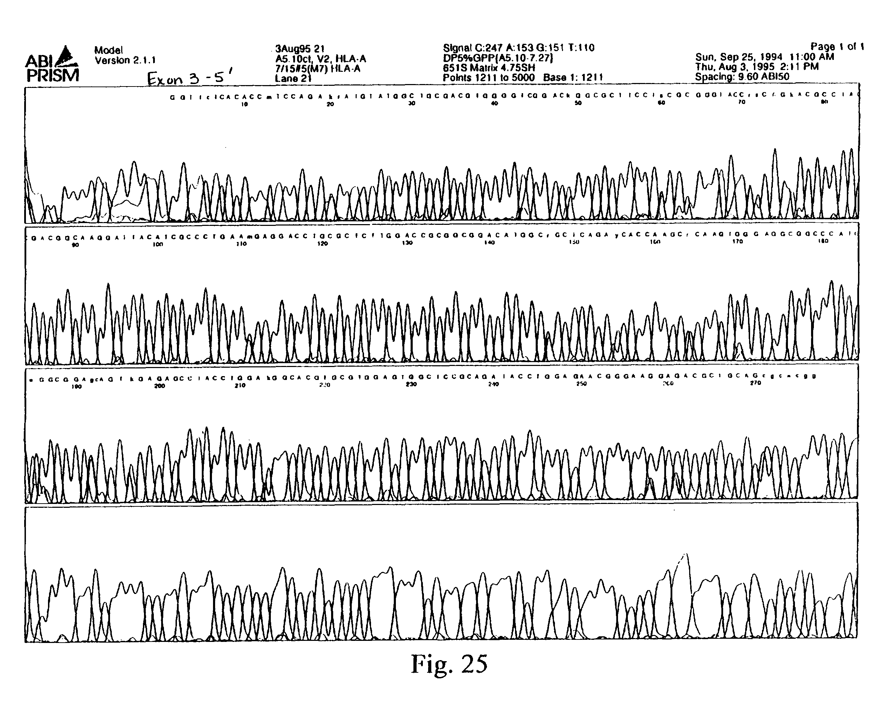

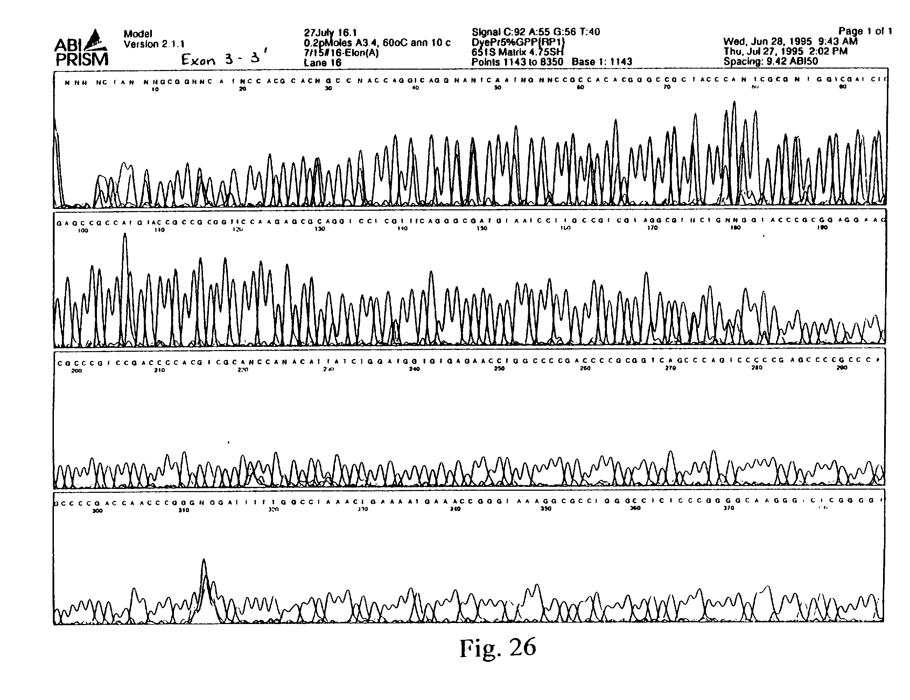

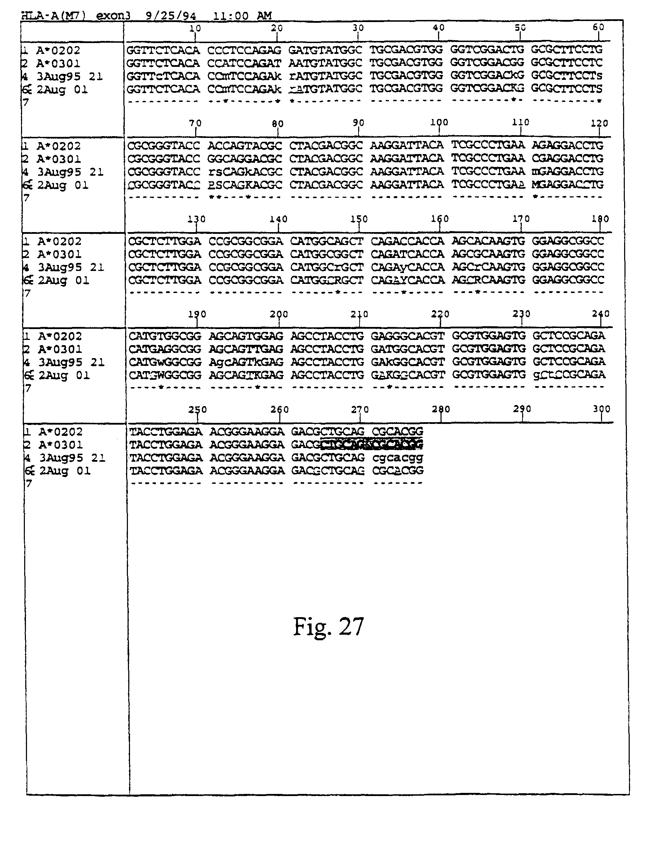

Figs. 25 and 26 show the raw sequence of the sense strand and the antisense strand of exon 3 of the HLA-A gene, respectively. Sequencing primers SEQ ID #27 and SEQ ID #28 were used to obtain the sequence of the sense strand shown in Fig. 25, and sequencing primer SEQ ID #29 was used to obtain the sequence of the antisense strand shown in Fig 26. Fig. 27 shows a sequence alignment of the data from Figs. 25 and 26 where: lines 1 and 2 are the reference sequence of the putative diploid alleles, HLA-A0202 and HLA-A0301, respectively; line 3 is the experimentally determined sequence of the sense strand obtained from Fig. 25; and line 4 is the experimentally determined sequence of the antisense strand obtained from Fig. 26 (note that the line numbers 1, 2, 3, and 4 refer to the numbers in the leftmost column of each of the sequence panels in Fig. 24).

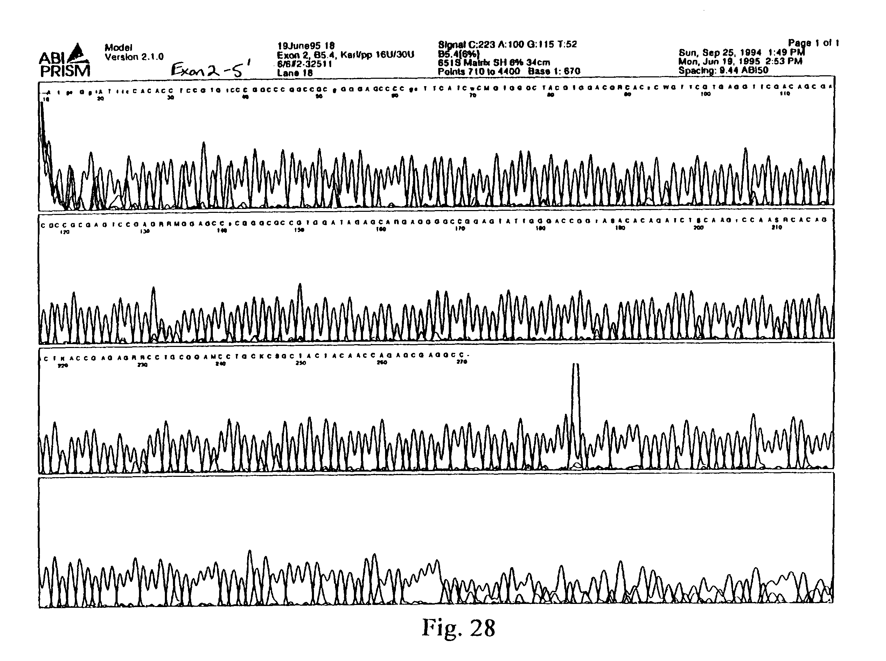

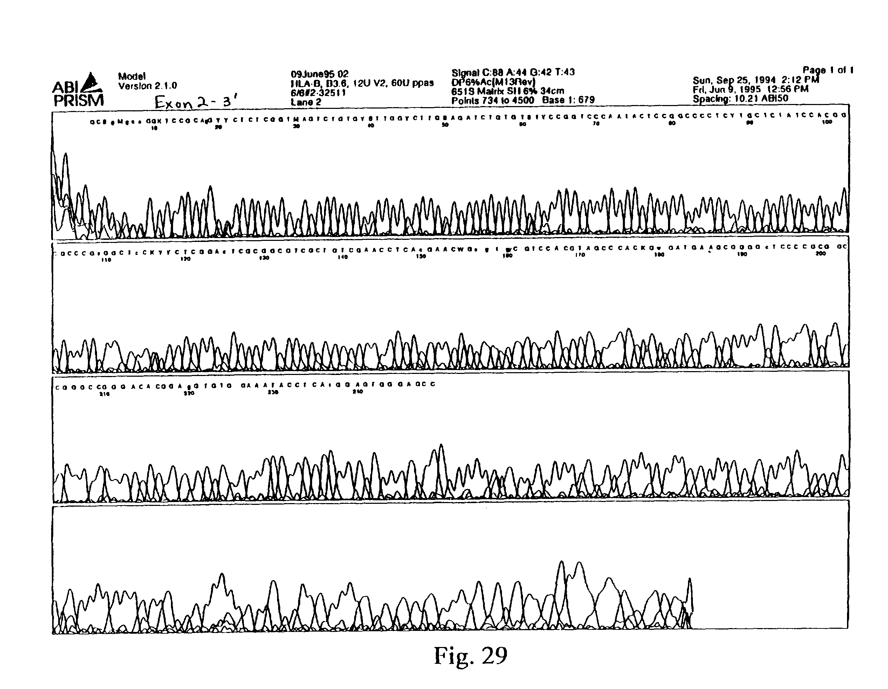

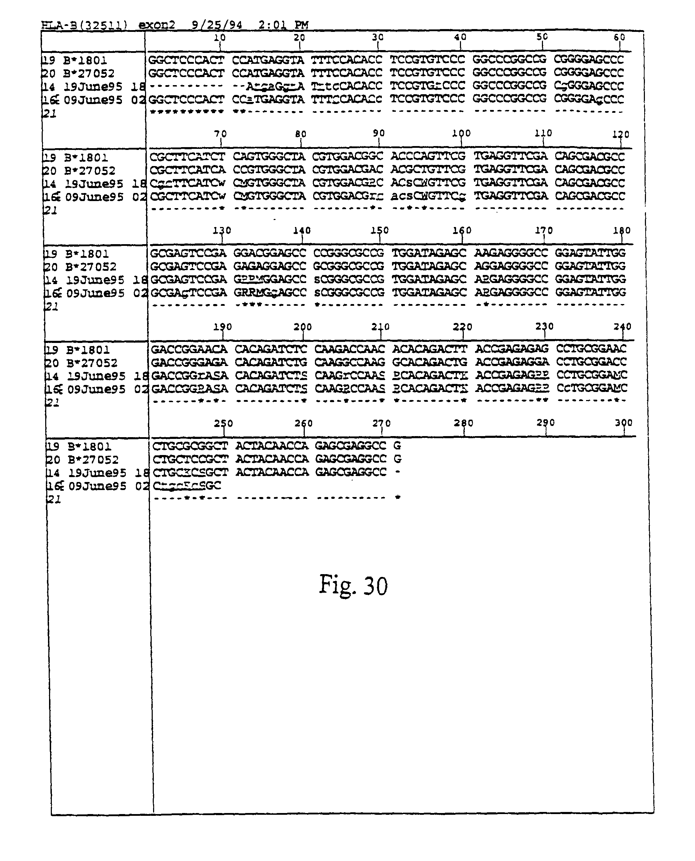

Figs. 28-33 show typing data from the HLA-B class I gene. Figs. 28 and 29 show the raw sequence of the sense strand and the antisense strand of exon 2 of the HLA-B gene, respectively. Sequencing primer SEQ ID #9 was used to obtain the sequence of the sense strand shown in Fig. 28, and sequencing primer SEQ ID #12 was used to obtain the sequence of the antisense strand shown in Fig 29. Fig. 30 shows a sequence alignment of the data from Figs. 28 and 29 where: lines 19 and 20 are the reference sequence of the putative diploid alleles, HLA-B-1801 and HLA-B27052, respectively; line 14 is the experimentally determined sequence of the sense strand obtained from Fig. 28; and line 16 is the experimentally determined sequence of the antisense strand obtained from Fig. 29 (note that the line numbers 19, 20, 14, and 16 refer to the numbers in the leftmost column of each of the sequence panels in Fig. 30).

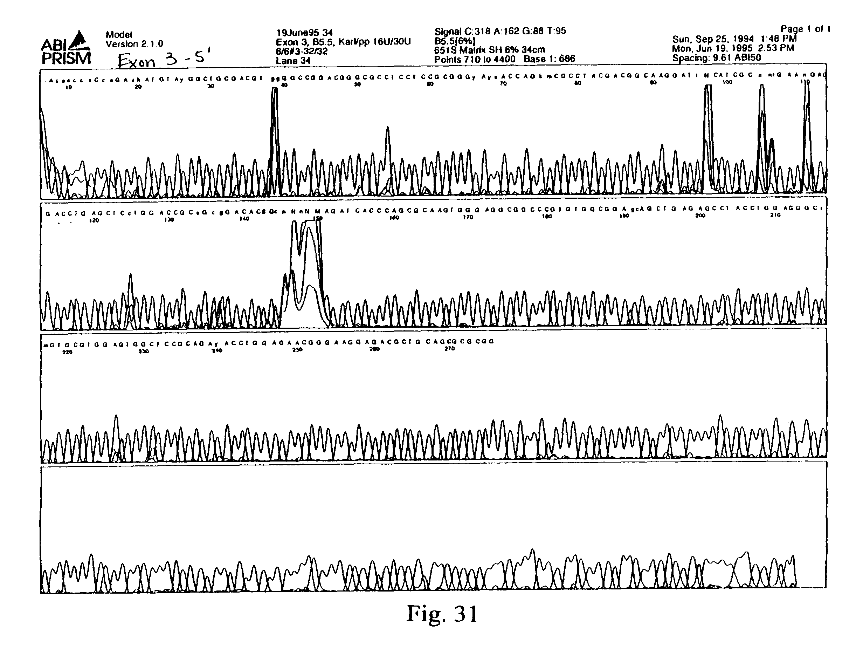

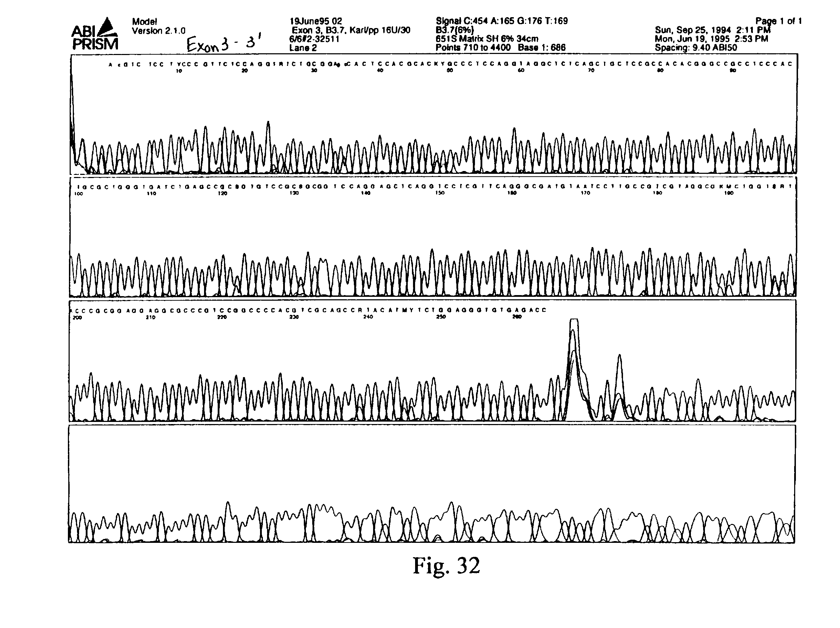

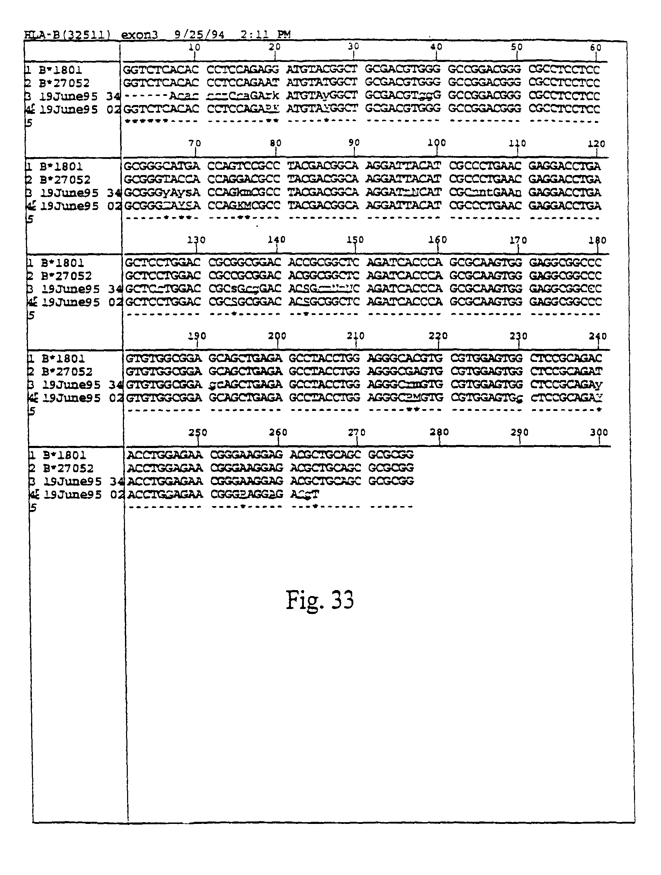

Figs. 31 and 32 show the raw sequence of the sense strand and the antisense strand of exon 3 of the HLA-B gene, respectively. Sequencing primer SEQ ID #10 was used to obtain the sequence of the sense strand shown in Fig. 31, and sequencing primer SEQ ID #13 was used to obtain the sequence of the antisense strand shown in Fig 32. Fig. 33 shows a sequence alignment of the data from Figs. 31 and 32 where: lines 1 and 2 are the reference sequence of the putative diploid alleles, HLA-B1801 and HLA-B27052, respectively; line 3 is the experimentally determined sequence of the sense strand obtained from Fig. 31; and line 4 is the experimentally determined sequence of the antisense strand obtained from Fig. 32 (note that the line numbers 1, 2, 3, and 4 refer to the numbers in the leftmost column of each of the sequence panels in Fig. 33).

### EXAMPLE 5

### PCR Amplification of HLA-C Class I Genes

PCR amplification was performed using a Perkin-Elmer 9600 thermal cycler. The amplification was performed with a wax-barrier hot start using Ampliwax™ wax pellets (PE p/n N808-0100). The amplification was performed on human genomic DNA that was purified from peripheral blood cells using the salting-out method, e.g., Miller et al., *supra.* The average length of the purified genomic DNA was estimated to be at least 5 kilobases.

The amplification primers used for amplifying the HLA-C gene are shown in Table VII below.

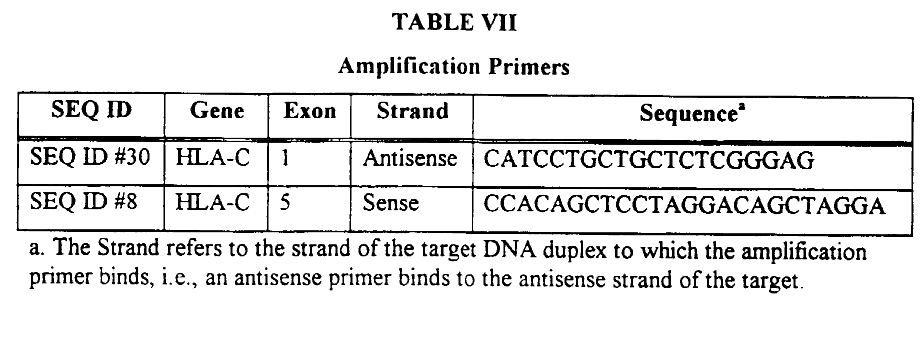

The PCR reaction for the HLA-C gene was prepared as follows. Five µl of a 10 mM deoxynucleotide triphosphate (dNTP) solution (2.5 mM each of dATP, dCTP, dGTP, and dTTP), 1 µl of a 10 pmole/ µl solution of the SEQ ID #13 amplification primer, 1 µl of a 10 pmole/ µl solution of the SEQ ID #8 amplification primer, a volume of sterile double-distilled (dd) H₂0 sufficient to result in a final volume of 25 µl.

Next, the above-prepared 25 µl reaction mixture was added to a Microamp reaction tube (PE p/n N801-0533, N801-0534, N801-0540) containing one Ampliwax™ pellet (PE p/n N808-0100). The tube was capped, briefly spun in a centrifuge at 3000 rpm to remove all droplets from the side of the tube, then heated to 90°C for 1 min to melt the Ampliwax™ pellet. The tube was then cooled to 4°C, thus forming a wax liquid barrier.

Above the wax barrier was added a solution containing 9.65 µl of ddH₂O, 0.35 µl of a 5 U/ µl solution of AmpliTaq™ DNA polymerase enzyme, 10 µl of the SX concentrated PCR buffer, and 5 µl of a 10 ng/ µl solution of purified genomic DNA, resulting in 25 µl total volume.

The above-prepared reaction tube was placed in a Perkin-Elmer 9600 thermal cycler, denatured at 98 °C for 20 s, then subjected to the following thermal cycle program: (i) 98 °C for 10 s, 65 °C for 2 min, where the cycle was repeated eight times; followed by (ii) 96 °C for 10 s, 65 °C for 2 min, where the cycle was repeated 32 times.

Following thermocycling, 10 µl of each of the reactions was analyzed by agarose gel electrophoresis to ensure proper PCR amplification. A 0.7% agarose gel was used containing ethidium bromide at 0.8 µg/ml and TBE buffer (89mM Tris-HCl, 89mM Boric Acid, 2mM Na₂EDTA, pH8.3) as both the gel and running buffer. The gel was electrophoresed at 7 V/cm for 1 hr and visualized using a UV transilluminator. A band at about 2.0 kb for the HLA-C product was seen indicating successful amplification of the specific gene (sizes based on internal size standards).

### EXAMPLE 6

### DNA Sequencing of Exons 2 and 3 of the HLA-C Class I Gene Using the Amplification Product from Example 5

Sequencing was performed with no purification of the PCR products using the AmpliTaq® DNA polymerase FS enzyme from Perkin-Elmer (p/n 402114). The sequencing protocol used was that suggested by Perkin-Elmer with some minor modifications, e.g., ABI Prism Dye Primer Cycle Sequencing Core Kit Protocol, Revision A, July 1995, *supra.* The sequencing primers shown below in Table VIII were used to sequence exons 2 and 3 of the HLA-C gene.

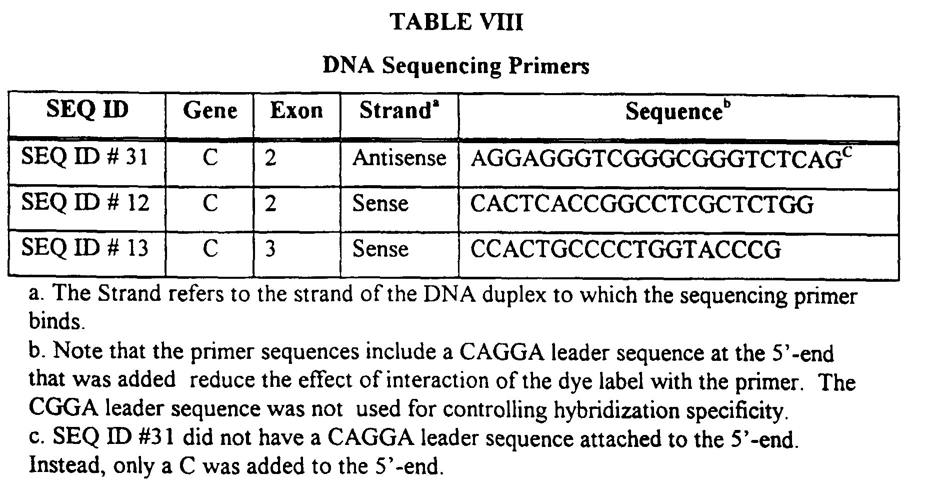

Each of the primers in Table VIII is labeled at the 5'-end with one of the four fluorescent dyes 5-carboxy-fluorescein (FAM), 2',7'-dimethoxy-4', 5'-dichloro-6-carboxy-fluorescene (JOE), N,N,N',N'-tetramethyl-6-carboxyrhodamine (TAMRA), and 6-carboxy-X-rhodamine (ROX), e.g., U.S. Pat. No. 4,855,225.

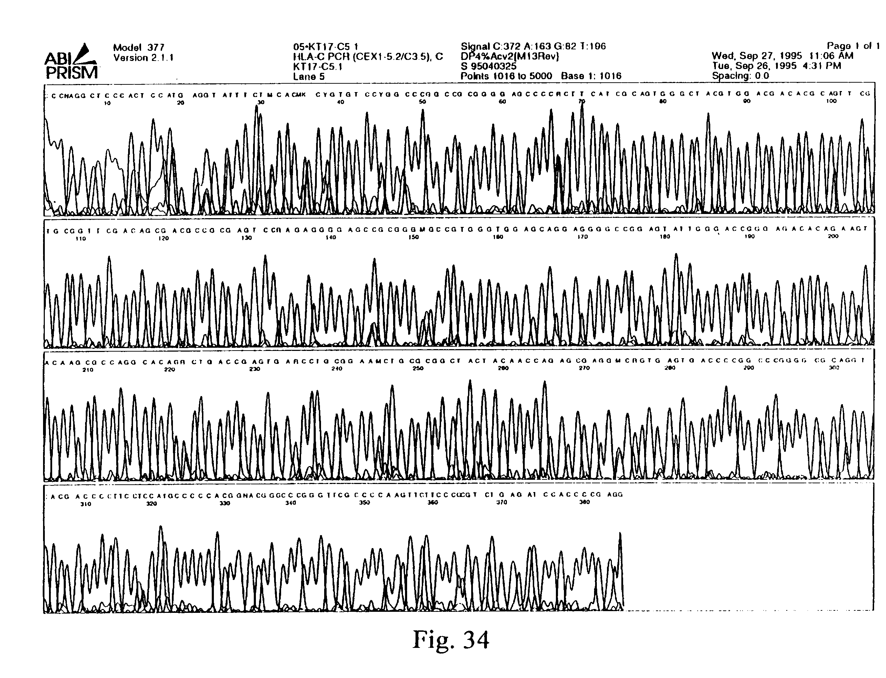

The following Figs. 34-38 show examples of HLA class I typing data collected using the protocols of Examples 5 and 6.

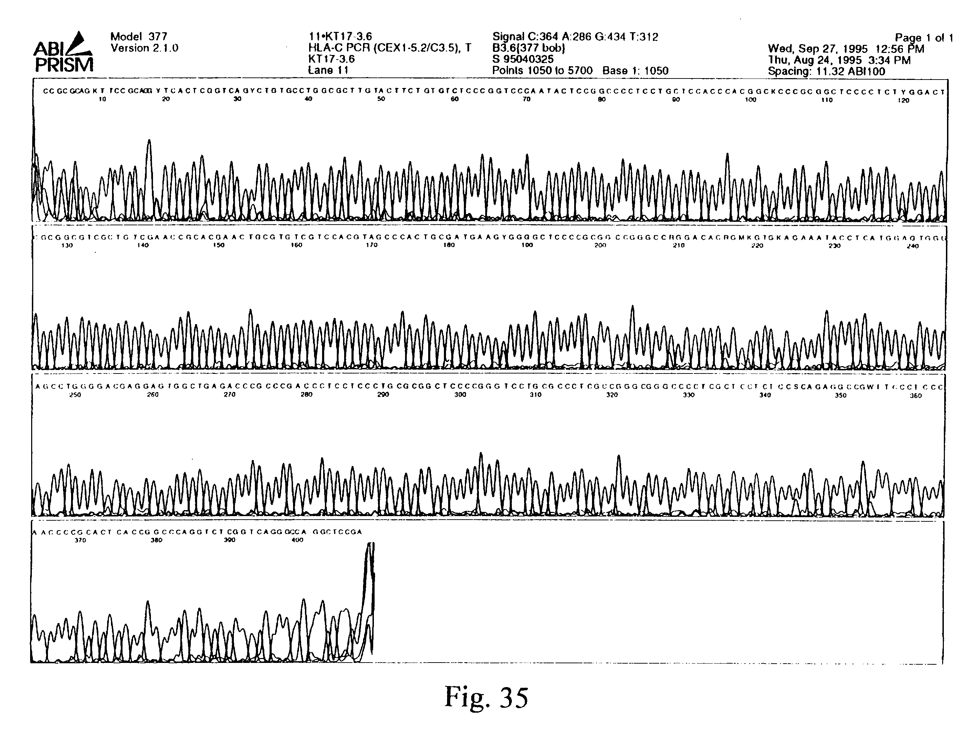

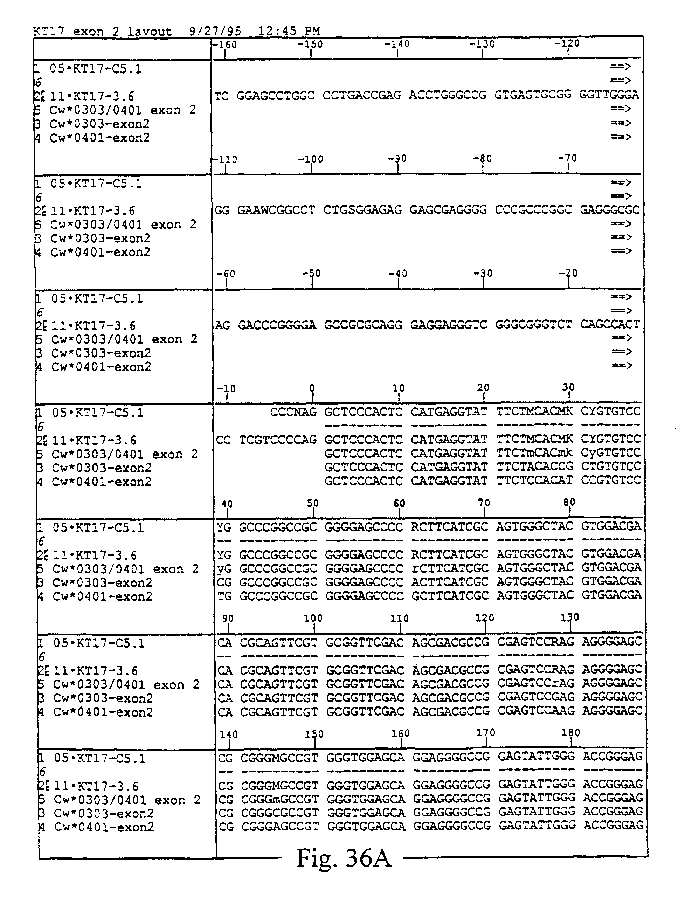

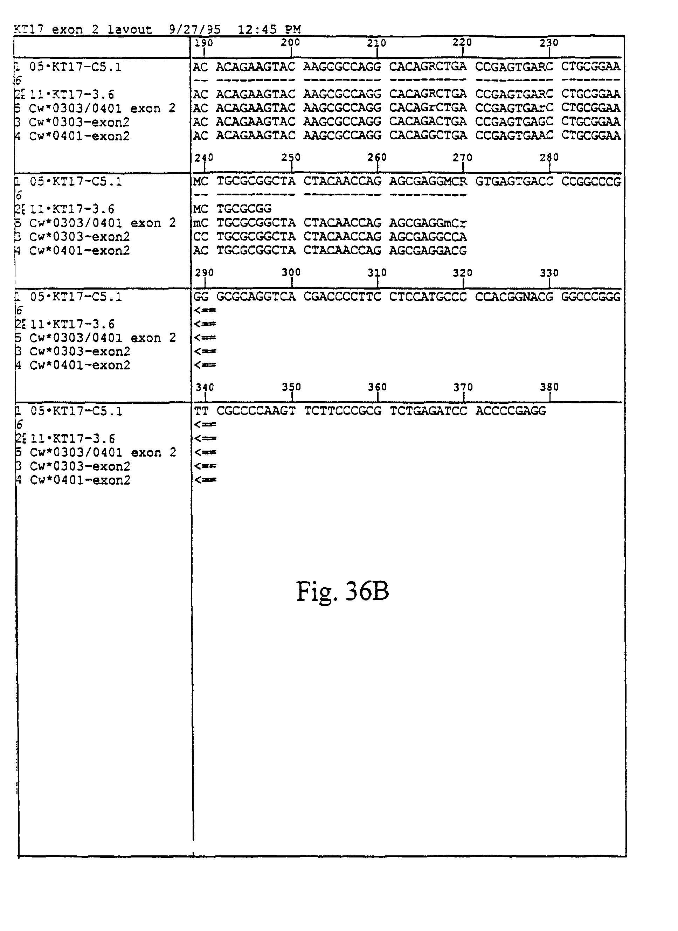

Figs. 34-38 show typing data from the HLA-C class I gene. Figs. 34 and 35 show the raw sequence of the sense strand and the antisense strand of exon 2 of the HLA-C gene, respectively. Sequencing primer SEQ ID #31 was used to obtain the sequence of the sense strand shown in Fig. 34, and sequencing primer SEQ ID #12 was used to obtain the sequence of the antisense strand shown in Fig 35. Figs. 36A and 36B show a sequence alignment of the data from Figs. 34 and 35 where: line 3 and 4 are the reference sequence of the putative diploid alleles, HLA-Cw0303 and HLA-Cw0401, respectively; lines 1 and 2 are the experimentally determined sequences of the sense strand obtained from Fig. 34 and the antisense strand obtained from Fig. 35; and line 5 is a consensus sequence for the heterozygote allele (note that the line numbers 1, 2, 3, 4 and 5 refer to the numbers in the leftmost column of each of the sequence panels in Figs. 36A and 36B).

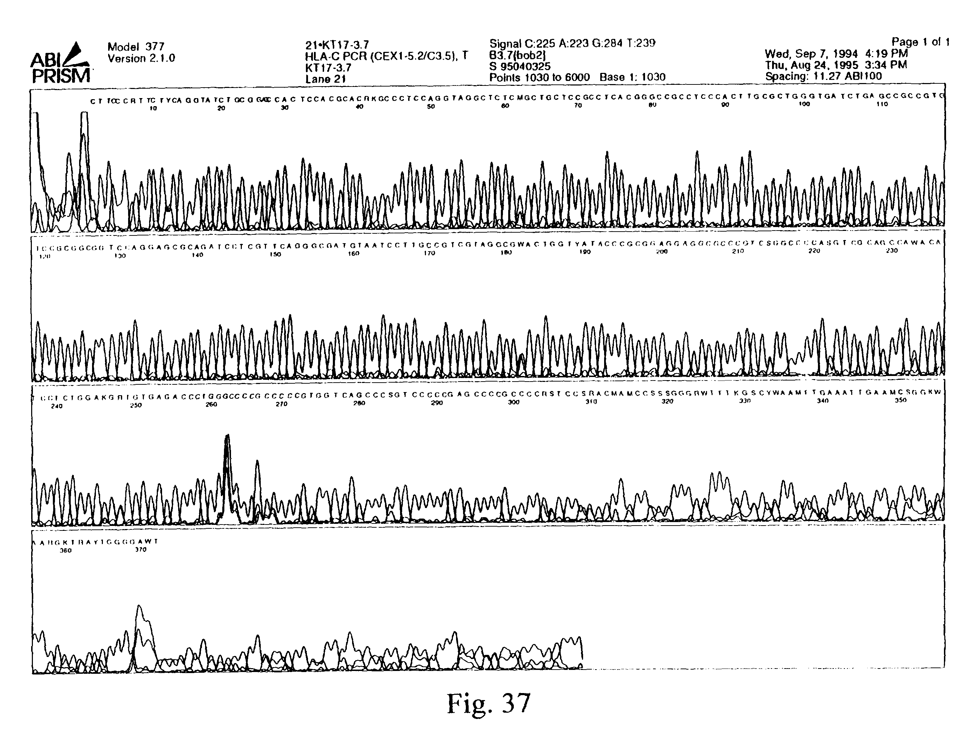

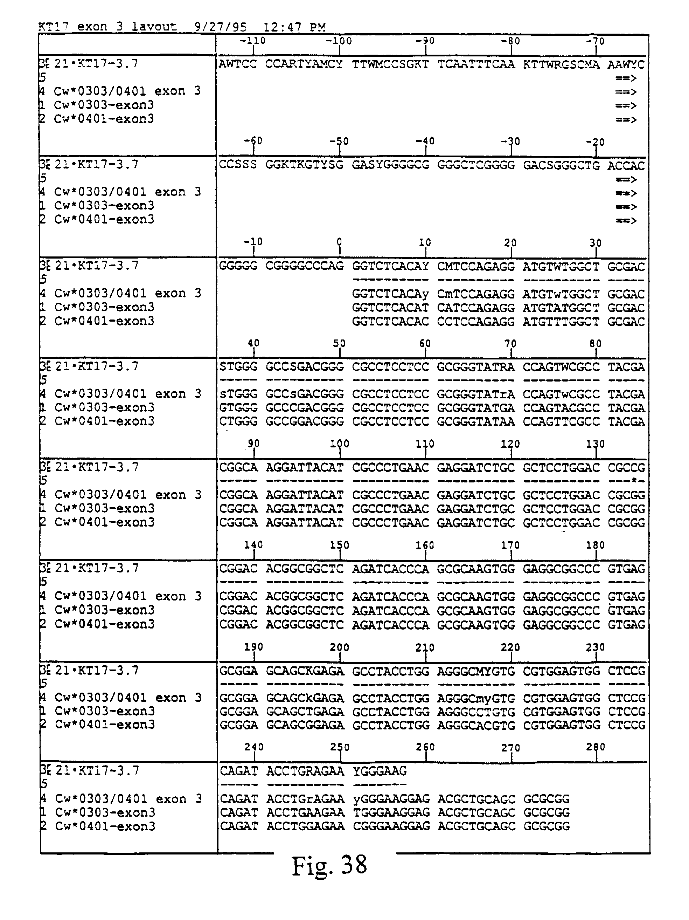

Fig. 37 shows the raw sequence of the antisense strand of exon 3 of the HLA-C gene. Sequencing primer SEQ ID #13 was used to obtain the sequence of the antisense strand shown in Fig. 37. Fig.38 shows a sequence alignment of the data from Fig. 37 where: lines 1 and 2 are the reference sequence of the putative diploid alleles, HLA-Cw0303 and HLA-Cw0401, respectively; line 3 is the experimentally determined sequence of the antisense strand obtained from Fig. 37; and line 4 is a consensus sequence for the heterozygote allele (note that the line numbers 1, 2, 3, and 4 refer to the numbers in the leftmost column of each of the sequence panels in Fig. 38).

## Claims

1. A method useful for typing HLA-A, -B, or C class I genes comprising the steps of:
providing a sample DNA containing a HLA-A, -B, or C class I gene having a first exon, a second exon, and a target sequence;
contacting the sample DNA with a first amplification primer, the first amplification primer including sequence complementary to sequence located in exon 1 of the HLA-A, -B, or C gene;
contacting the sample DNA with a second amplification primer, the second amplification primer including sequence complementary to sequence located in exon 5 of the HLA-A, -B, or C gene;
amplifying the target sequence of the sample DNA by PCR using the first and second amplification primers, thereby forming an amplicon; and
detecting the amplicon using a sequence-specific detection method.

2. The method of **claim 1** wherein the HLA class I gene is a HLA-A gene and the first amplification primer comprises the sequence CGCCGAGGATGGCCGTC (SEQ ID #1) and the second amplification primer comprises the degenerate sequences GGAGAACCAGGCCAGCAATGATGCCC (SEQ ID #2) and GGAGAACTAGGCCAGCAATGATGCCC (SEQ ID #3).

3. The method of **claim 1** wherein the HLA class I gene is a HLA-B gene and the first amplification primer comprises the degenerate sequences CCTCCTGCTGCTCTCGGC (SEQ ID #21) and CCTCCTGCTGCTCTCGGGA (SEQ ID#22), and GCTGCTCTGGGGGGCAG (SEQ ID #23), and the second amplification primer comprises the sequence GCTCCGATGACCACAACTGCT (SEQ ID #24).

4. The method of **claim 1** wherein the HLA class I gene is a HLA-B gene and the first amplification primer comprises the sequence GGCCCTGACCGAGACCTGG (SEQ ID #4) and the second amplification primer comprises the sequence TCCGATGACCACAACTGCTAGGAC (SEQ ID #5).

5. The method of **claim 1** wherein the HLA class I gene is a HLA-C gene and the first amplification primer comprises the degenerate sequences GGCCCTGACCGAGACCTGGGC (SEQ ID #6) and GGCCCTGACCCAGACCTGGGC (SEQ ID #7) and the second amplification primer comprises the sequence CCACAGCTCCTAGGACAGCTAGGA (SEQ ID #8).

6. The method of **claim 1** wherein the HLA class I gene is a HLA-C gene and the first amplification primer comprises the sequence CATCCTGCTGCTCTCGGGAG (SEQ ID #30) and the second amplification primer comprises the sequence CCACAGCTCCTAGGACAGCTAGGA (SEQ ID #8).

7. The method of **claim 1** wherein the sequence-specific detection method is DNA sequencing.

8. The method of **claim 7** wherein the DNA sequencing method is Sanger-type DNA sequencing comprising the steps of:
contacting the amplicon with a sequencing primer;
extending the sequencing primer using a polymerase in the presense of deoxynucleotides and dideoxynucleotides, thereby forming a mixture of extension products differing in length;
separating the extension products such that extension products differing in length by a single nucleotide are resolved; and,
detecting the separated extension products.

9. The method of **claim 8** wherein the sequencing primer (i) is between 10 nucleotides and 30 nucleotides in length and (ii) has a nucleotide sequence complementary to the HLA-A, HLA-B, and HLA-C genes in the nucleotide sequence regions selected from the group consisting of:
a first nucleotide sequence region from -20 nucleotides to +20 nucleotides of the 5' intron-exon border of the sense strand of exon 2;
a second nucleotide sequence region from -30 nucleotides to +20 nucleotides of the 5' intron-exon border of the sense strand of exon 3;
a third nucleotide sequence region from -30 nucleotides to +20 nucleotides of the 5' intron-exon border of the sense strand exon 4;
a fourth nucleotide sequence region from +30 nucleotides to -20 nucleotides of the 5' intron-exon border of the antisense strand exon 2;
a fifth nucleotide sequence region from +20 nucleotides to -20 nucleotides of the 5' intron-exon border of the antisense strand of exon 3; and
a sixth nucleotide sequence region from +40 nucleotides to -10 nucleotides of the 5' intron-exon border of the antisense strand of exon 4.

10. The method of **claim 9** wherein each member of the set of sequencing primers is selected from the group consisting of:
a first sequencing primer having the sequence CTCGCCCCCAGGCTCCCAC (SEQ ID #15);
a second sequencing primer having the sequence GCGGGGGCGGGTCCAGG (SEQ ID #16);
a third sequencing primer having the sequence CTGACTCTTCCCATCAGACCC (SEQ ID #17);
a fourth sequencing primer having the sequence CACTCACCGGCCTCGCTCTGG (SEQ ID #18);
a fifth sequencing primer having the sequence CCACTGCCCCTGGTACCCG (SEQ ID #19); and
a sixth sequencing primer having the sequence AGGGTGAGGGCTTCGGCAGCC (SEQ ID #20).

11. The method of **claim 8** wherein the HLA class 1 gene is the sense strand of exon 2 of the HLA-A gene and the sequencing primer comprises the degenerate sequences TCGGGCAGGTCTCAGCC (SEQ ID#25) and TCGGGCGGGTCTCAGCC (SEQ ID #26).

12. The method of **claim 8** wherein the HLA class 1 gene is the antisense strand of exon 2 of the HLA-A gene and the sequencing primer comprises the sequence CACTCACCGGCCTCGCTCTGG (SEQ ID #12).

13. The method of **claim 8** wherein the HLA class 1 gene is the sense strand of exon 3 of the HLA-A gene and the sequencing primer comprises the degenerate sequences GGGCTCGGGGGACCGGG (SEQ ID #27) and GGGCTCGGGGGACTGGG (SEQ ID #28).

14. The method of **claim 8** wherein the HLA class 1 gene is the sense strand of exon 2 of the HLA-B gene and the sequencing primer comprises the sequence CTCGCCCCCAGGCTCCCAC (SEQ ID #9).

15. The method of **claim 8** wherein the HLA class 1 gene is the antisense strand of exon 2 of the HLA-B gene and the sequencing primer comprises the sequence CACTCACCGGCCTCGCTCTGG (SEQ ID #12).

16. The method of **claim 8** wherein the HLA class 1 gene is the sense strand of exon 3 of the HLA-B gene and the sequencing primer comprises the sequence GCGGGGGCGGGTCCAGG (SEQ ID #10).

17. The method of **claim 8** wherein the HLA class 1 gene is the antisense strand of exon 3 of the HLA-B gene and the sequencing primer comprises the sequence CCACTGCCCCTGGTACCCG (SEQ ID #13).

18. The method of **claim 8** wherein the HLA class 1 gene is the sense strand of exon 2 of the HLA-C gene and the sequencing primer comprises the sequence AGGAGGGTCGGGCGGGTCTCAG (SEQ ID #31).

19. The method of **claim 8** wherein the HLA class 1 gene is the antisense strand of exon 2 of the HLA-C gene and the sequencing primer comprises the sequence CACTCACCGGCCTCGCTCTGG (SEQ ID #12).

20. The method of **claim 8** wherein the HLA class 1 gene is the antisense strand of exon 3 of the HLA-C gene and the sequencing primer comprises the sequence CCACTGCCCCTGGTACCCG (SEQ ID #13).

21. The method of **claim 1** further comprising the step of comparing the determined DNA sequence with the DNA sequences of known HLA types.

22. A kit useful for typing HLA-A, -B, or -C class I genes comprising:
a thermostable polymerase;
each of the A, G, C, and T deoxynucleotides;
a buffer; and
first and second amplification primers, each amplification primer being between 10 nucleotides and 30 nucleotides in length, the first primer having a nucleotide sequence complementary to sequence located in exon 1 of the HLA-A, -B, or -C gene, and the second primer having a nucleotide sequence complementary to sequence located in exon 5 of the HLA-A, -B, or -C gene.

23. The kit of **claim 22** further comprising:
a sequencing reagent comprising:
a polymerase;
each of the A, G, C, and T deoxynucleotides;
each of the A, G, C, and T dideoxydeoxynucleotides;
a buffer, and
a set of sequencing primers wherein each member of the set of sequencing primers (i) is between 10 nucleotides and 30 nucleotides in length and (ii) has a nucleotide sequence complementary to the HLA-A HLA-B, and HLA-C genes in the nucleotide sequence regions selected from the group consisting of:
a first nucleotide sequence region from -20 nucleotides to +20 nucleotides of the 5' intron-exon border of the sense strand of exon 2,
a second nucleotide sequence region from -30 nucleotides to +20 nucleotides of the 5' intron-exon border of he sense strand of exon 3;
a third nucleotide sequence region from -30 nucleotides to +20 nucleotides of the 5' intron-exon border of the sense strand exon 4;
a fourth nucleotide sequence region from +30 nucleotides to -20 nucleotides of the 5' intron-exon border of the antisense strand exon 2;
a fifth nucleotide sequence region from +20 nucleotides to -20 nucleotides of the 5' intron-exon border of the antisense strand of exon 3; and
a sixth nucleotide sequence region from +40 nucleotides to -10 nucleotides of the 5' intron-exon border of the antisense strand of exon 4.

24. PCR amplification primers useful for amplification of the HLA-A class 1 gene comprising:
a first primer comprising the sequence CGCCGAGGATGGCCGTC (SEQ ID #1); and
a second primer comprising the degenerate sequences GGAGAACCAGGCCAGCAATGATGCCC (SEQ ID #2) and GGAGAACTAGGCCAGCAATGATGCCC (SEQ ID #3).

25. PCR amplification primers useful for amplification of the HLA-B class 1 gene selected from the group consisting of
a first set comprising:
a first primer comprising the sequence GGCCCTGACCGAGACCTGG (SEQ ID #4); and
a second primer comprising the sequence TCCGATGACCACAACTGCTAGGAC (SEQ ID #5); and
a second set comprising:
a first primer comprising the degenerate sequences CCTCCTGCTGCTCTCGGC (SEQ ID #21), and CCTCCTGCTGCTCTCGGGA (SEQ ID #22), and GCTGCTCTGGGGGGCAG (SEQ ID #23); and
a second primer comprising the sequence GCTCCGATGACCACAACTGCT (SEQ ID #24).

26. PCR amplification primers useful for amplification of the HLA-C class 1 gene selected from the group consisting of:
a first set comprising:
a first primer comprising the degenerate sequences GGCCCTGACCGAGACCTGGGC (SEQ ID #6), and GGCCCTGACCCAGACCTGGGC (SEQ ID #7); and
a second primer comprising the sequence CCACAGCTCCTAGGACAGCTAGGA (SEQ ID #8); and
a second set comprising:
a first primer comprising the sequence CATCCTGCTGCTCTCGGGAG (SEQ ID #30); and
a second primer comprising the sequence CCACAGCTCCTAGGACAGCTAGGA (SEQ ID #8).

27. A sequencing primer useful for sequencing exon 2 of HLA-A, -B, or -C class 1 genes comprising a sequence selected from the group consisting of the sequence CTCGCCCCCAGGCTCCCAC (SEQ ID #9); the sequence CACTCACCGGCCTCGCTCTGG (SEQ ID #12); the degenerate sequences TCGGGCAGGTCTCAGCC (SEQ ID #25) and TCGGGCGGGTCTCAGCC (SEQ ID #26); and the sequence AGGAGGGTCGGGCGGGTCTCAG(SEQ ID #31).

28. A sequencing primer useful for sequencing exon 3 of HLA-A, -B, or -C class 1 genes comprising a sequence selected from the group consisting of the sequence GCGGGGGCGGGTCCAGG (SEQ ID #10); the sequence CCACTGCCCCTGGTACCCG (SEQ ID #13); the sequence GAGGCGCCCCGTGGC (SEQ ID #29); the degenerate sequences GGGCTCGGGGGACCGGG (SEQ ID #27) and GGGCTCGGGGGACTGGG (SEQ ID #28); and the sequence GGGCTGACCACGGGGGCGGGGCCCAG (SEQ ID #32).

29. A sequencing primer useful for sequencing exon 4 of HLA-A, -B, or -C class 1 genes comprising a sequence selected from the group consisting of the sequence CTGACTCTTCCCATCAGACCC (SEQ ID #11); and the sequence AGGGTGAGGGGCTTCGGCAGCC (SEQ ID #14).

## Patentansprüche

1. Ein Verfahren zur Typisierung von HLA-A, -B, oder -C Klasse I-Genen, umfassend die Schritte:
Bereitstellen einer Proben-DNA, die ein HLA-A, -B, oder -C Klasse I-Gen mit einem ersten Exon, einem zweiten Exon und eine Zielsequenz enthält;
in Kontakt bringen der Proben-DNA mit einem ersten Amplifizierungsprimer, wobei der erste Amplifizierungsprimer eine Sequenz einschliesst, die zu einer in Exon 1 des HLA-A, -B oder -C-Gens gelegenen Sequenz komplementär ist;
in Kontakt bringen der Proben-DNA mit einem zweiten Amplifizierungsprimer, wobei der zweite Amplifizierungsprimer eine Sequenz einschliesst, die zu einer in Exon 5 des HLA-A, -B oder C-Gens gelegenen Sequenz komplementär ist;
Amplifizieren der Zielsequenz der Proben-DNA mittels PCR unter Verwendung des ersten und zweiten Amplifizierungsprimers unter Bildung eines Amplikons; und
Nachweis des Amplikons unter Verwendung einer Sequenz-spezifischen Methode.

2. Verfahren gemäß Anspruch 1, wobei das HLA-Klasse I Gen ein HLA-A Gen ist und der erste Amplifizierungsprimer die Sequenz CGCCGAGGATGGCCGTC (SEQ ID#1) und der zweite Amplifizierungsprimer die degenerierten Sequenzen GGAGAACCAGGCCAGCAATGATGCCC (SEQ ID #2) und GGAGAACTAGGCCAGCAATGATGCCC (SEQ ID #3) umfasst.

3. Verfahren gemäß Anspruch 1, wobei das HLA-Klasse I Gen ein HLA-B Gen ist und der erste Amplifizierungsprimer die degenerierten Sequenzen CCTCCTGCTGCTCTCGGC (SEQ ID #21) und CCTCCTGCTGCTCTCGGGA (SEQ ID #22) und GCTGCTCTGGGGGGCAG (SEQ ID #23) und der zweite Amplifizierungsprimer die Sequenz GCTCCGATGACCACAACTGCT (SEQ ID #24) umfasst.

4. Verfahren gemäß Anspruch 1, wobei das HLA-Klasse I Gen ein HLA-B Gen ist und der erste Amplifizierungsprimer die Sequenz GGCCCTGACCGAGACCTGG (SEQ ID #4) und der zweite Amplifizierungsprimer die Sequenz TCCGATGACCACAACTGCTAGGAC (SEQ ID #5) umfasst.

5. Verfahren gemäß Anspruch 1, wobei das HLA-Klasse I Gen ein HLA-C Gen ist und der erste Amplifizierungsprimer die degenerierten Sequenzen GGCCCTGACCGAGACCTGGGC (SEQ ID #6) und GGCCCTGACCCAGACCTGGGC (SEQ ID #7) und der zweite Amplifizierungsprimer die Sequenz CCACAGCTCCTAGGACAGCTAGGA (SEQ ID #8) umfasst.

6. Verfahren gemäß Anspruch 1, wobei das HLA-Klasse 1 Gen ein HLA-C Gen ist und der erste Amplifizierungsprimer die Sequenz CATCCTGCTGCTCTCGGGAG (SEQ ID #30) und der zweite Amplifizierungsprimer die Sequenz CCACAGCTCCTAGGACAGCTAGGA (SEQ ID #8) umfasst.

7. Verfahren gemäß Anspruch 1, wobei die Sequenz-spezifische Nachweismethode DNA-Sequenzierung ist.

8. Verfahren gemäß Anspruch 7, wobei das DNA-Sequenzierverfahren die Sanger-DNA-Sequenzierung ist, umfassend die Schritte:
in Kontakt bringen des Amplikons mit einem Sequenzierprimer;
Verlängern des Sequenzierprimers unter Verwendung einer Polymerase in Anwesenheit von Desoxynukleotiden und Didesoxynukleotiden unter Bildung eines Gemisches von Verlängerungsprodukten mit verschiedener Länge;
Auftrennen der Verlängerungsprodukte, so daß Verlängerungsprodukte, die sich in der Länge um ein Nukleotid unterscheiden, aufgetrennt werden; und
Nachweisen der aufgetrennten Verlängerungsprodukte.

9. Verfahren gemäß Anspruch 8, wobei der Sequenzierprimer (i) zwischen 10 und 30 Nukleotiden lang ist und (ii) eine Nukleotidsequenz besitzt, die komplementär zu den HLA-A, HLA-B, und HLA-C Genen in den Nukleotidsequenz-Regionen ist, ausgewählt aus der Gruppe bestehend aus:
einer ersten Nukleotidsequenz-Region von -20 Nukleotiden bis +20 Nukleotiden der 5'-Intron-Exon-Grenze des Sense-Stranges von Exon 2;
einer zweiten Nukleotidsequenz-Region von -30 Nukleotiden bis +20 Nukleotiden der 5'-Intron-Exon-Grenze des Sense-Stranges von Exon 3;
einer dritten Nukleotidsequenz-Region von -30 Nukleotiden bis +20 Nukleotiden der 5'-Intron-Exon-Grenze des Sense-Stranges von Exon 4;
einer vierten Nukleotidsequenz-Region von +30 Nukleotiden bis -20 Nukleotiden der 5'-Intron-Exon-Grenze des Antisense-Stranges von Exon 2;
einer fünften Nukleotidsequenz-Region von +20 Nukleotiden bis -20 Nukleotiden der 5'-Intron-Exon-Grenze des Antisense-Stranges von Exon 3; und
einer sechsten Nukleotidsequenz-Region von +40 Nukleotiden bis -10 Nukleotiden der 5'-Intron-Exon-Grenze des Antisense-Stranges von Exon 4.

10. Verfahren gemäß Anspruch 9, wobei jedes Mitglied des Satzes von Sequenzierprimern ausgewählt ist aus der Gruppe bestehend aus:
einem ersten Sequenzierprimer mit der Sequenz CTCGCCCCCAGGCTCCCAC (SEQ ID #15);
einem zweiten Sequenzierprimer mit der Sequenz GCGGGGGCGGGTCCAGG (SEQ ID #16);
einem dritten Sequenzierprimer mit der Sequenz CTGACTCTTCCCATCAGACCC (SEQ ID #17);
einem vierten Sequenzierprimer mit der Sequenz CACTCACCGGCCTCGCTCTGG (SEQ ID #18);
einem fünften Sequenzierprimer mit der Sequenz CCACTGCCCCTGGTACCCG (SEQ ID #19); und
einem sechsten Sequenzierprimer mit der Sequenz AGGGTGAGGGGCTTCGGCAGCC (SEQ ID #20).

11. Verfahren gemäß Anspruch 8, wobei das HLA Klasse I Gen der Sense-Strang von Exon 2 des HLA-A-Gens ist und der Sequenzierprimer die degenerierten Sequenzen TCGGGCAGGTCTCAGCC (SEQ ID #25) und TCGGGCGGGTCTCAGCC (SEQ ID #26) umfasst.

12. Verfahren gemäß Anspruch 8, wobei das HLA Klasse I Gen der Antisense-Strang von Exon 2 des HLA-A-Gens ist und der Sequenzierprimer die Sequenz CACTCACCGGCCTCGCTCTGG (SEQ ID #12) umfasst.

13. Verfahren gemäß Anspruch 8, wobei das HLA Klasse I Gen der Sense-Strang von Exon 3 des HLA-A-Gens ist und der Sequenzierprimer die degenerierten Sequenzen GGGCTCGGGGGACCGGG (SEQ ID #27) und GGGCTCGGGGGACTGGG (SEQ ID #28) umfasst.

14. Verfahren gemäß Anspruch 8, wobei das HLA Klasse I Gen der Sense-Strang von Exon 2 des HLA-B-Gens ist und der Sequenzierprimer die Sequenz CTCGCCCCCAGGCTCCCAC (SEQ ID #9) umfasst.

15. Verfahren gemäß Anspruch 8, wobei das HLA Klasse 1 Gen der Antisense-Strang von Exon 2 des HLA-B-Gens ist und der Sequenzierprimer die Sequenz CACTCACCGGCCTCGCTCTGG (SEQ ID #12) umfasst.

16. Verfahren gemäß Anspruch 8, wobei das HLA Klasse 1 Gen der Sense-Strang von Exon 3 des HLA-B-Gens ist und der Sequenzierprimer die Sequenz GCGGGGGCGGGTCCAGG (SEQ ID #10) umfasst.

17. Verfahren gemäß Anspruch 8, wobei das HLA Klasse I Gen der Antisense-Strang von Exon 3 des HLA-B-Gens ist und der Sequenzierprimer die Sequenz CCACTGCCCCTGGTACCCG (SEQ ID #13) umfasst.

18. Verfahren gemäß Anspruch 8, wobei das HLA Klasse I Gen der Sense-Strang von Exon 2 des HLA-C-Gens ist und der Sequenzierprimer die Sequenz AGGAGGGTCGGGCGGGTCTCAG (SEQ ID #31) umfasst.

19. Verfahren gemäß Anspruch 8, wobei das HLA Klasse 1 Gen der Antisense-Strang von Exon 2 des HLA-C-Gens ist und der Sequenzierprimer die Sequenz CACTCACCGGCCTCGCTCTGG (SEQ ID #12) umfasst.

20. Verfahren gemäß Anspruch 8, wobei das HLA Klasse I Gen der Antisense-Strang von Exon 3 des HLA-C-Gens ist und der Sequenzierprimer die Sequenz CCACTGCCCCTGGTACCCG (SEQ ID #13) umfasst.

21. Verfahren gemäß Anspruch 1, weiter umfassend den Schritt des Vergleichens der ermittelten DNA-Sequenz mit den DNA-Sequenzen bekannter HLA-Typen.

22. Ein Kit, nützlich zur Typisierung von HLA-A, -B, oder -C Klasse 1-Genen, umfassend:
eine thermostabile Polymerase;
jedes der A, G, C, und T-Desoxynukleotide;
einen Puffer; und
erste und zweite Amplifizierungsprimer, wobei jeder Amplifizierungsprimer zwischen 10 Nukleotiden und 30 Nukleotiden lang ist, wobei der erste Primer eine Nukleotidsequenz besitzt, die komplementär zu einer in Exon 1 des HLA-A, -B, oder -C-Gens gelegenen Sequenz ist, und der zweite Primer eine Nukleotidsequenz besitzt, die komplementär zu einer in Exon 5 des HLA-A, -B, oder -C-Gens gelegenen Sequenz ist.

23. Kit gemäß Anspruch 22, weiter umfassend:
ein Sequenzierreagenz, umfassend:
eine Polymerase;
jedes der A, G, C und T-Desoxnukleotide;
jedes der A, G, C und T-Didesoxynukleotide;
einen Puffer, und
einen Satz von Sequenzierprimern, wobei jedes Mitglied des Satzes von Sequenzierprimern (i) zwischen 10 Nukleotiden und 30 Nukleotiden lang ist und (ii) eine Nukleotidsequenz besitzt, die komplementär zu den HLA-A, HLA-B, und HLA-C Genen in den Nukleotidsequenz-Regionen ist, ausgewählt aus der Gruppe bestehend aus:
einer ersten Nukleotidsequenz-Region von -20 Nukleotiden bis +20 Nukleotiden der 5'-Intron-Exon-Grenze des Sense-Stranges von Exon 2;
einer zweiten Nukleotidsequenz-Region von -30 Nukleotiden bis +20 Nukleotiden der 5'-Intron-Exon-Grenze des Sense-Stranges von Exon 3;
einer dritten Nukleotidsequenz-Region von -30 Nukleotiden bis +20 Nukleotiden der 5'-Intron-Exon-Grenze des Sense-Stranges von Exon 4;
einer vierten Nukleotidsequenz-Region von +30 Nukleotiden bis -20 Nukleotiden der 5'-Intron-Exon-Grenze des Antisense-Stranges von Exon 2;
einer fünften Nukleotidsequenz-Region von +20 Nukleotiden bis -20 Nukleotiden der 5'-Intron-Exon-Grenze des Antisense-Stranges von Exon 3; und
einer sechsten Nukleotidsequenz-Region von +40 Nukleotiden bis -10 Nukleotiden der 5'-Intron-Exon-Grenze des Antisense-Stranges von Exon 4.

24. PCR-Amplifizierungsprimer zur Amplifizierung des HLA-A Klasse I-Gens, umfassend:
einen ersten Primer, umfassend die Sequenz CGCCGAGGATGGCCGTC (SEQ ID#1) und
einen zweiten Primer, umfassend die degenerierten Sequenzen GGAGAACCAGGCCAGCAATGATGCCC (SEQ ID #2) und GGAGAACTAGGCCAGCAATGATGCCC (SEQ ID #3).

25. PCR-Amplifizierungsprimer zur Amplifizierung des HLA-B Klasse 1-Gens, ausgewählt aus der Gruppe bestehend aus:
einem ersten Satz, umfassend:
einen ersten Primer, umfassend die Sequenz GGCCCTGACCGAGACCTGG (SEQ ID#4); und
einen zweiten Primer, umfassend die Sequenz TCCGATGACCACAACTGCTAGGAC (SEQ ID #5); und
einem zweiten Satz, umfassend:
einen ersten Primer, umfassend die degenerierten Sequenzen CCTCCTGCTGCTCTCGGC (SEQ ID #21), und CCTCCTGCTGCTCTCGGGA (SEQ ID #22) und GCTGCTCTGGGGGGCAG (SEQ ID #23); und
einen zweiten Primer, umfassend die Sequenz GCTCCGATGACCACAACTGCT (SEQ ID #24).

26. PCR-Amplifizierungsprimer zur Amplifizierung des HLA-C Klasse I-Gens, ausgewählt aus der Gruppe bestehend aus:
einem ersten Satz, umfassend:
einen ersten Primer, umfassend die degenerierten Sequenzen GGCCCTGACCGAGACCTGGGC (SEQ ID #6) und GGCCCTGACCCAGACCTGGGC (SEQ ID #7); und
einen zweiten Primer, umfassend die Sequenz CCACAGCTCCTAGGACAGCTAGGA (SEQ ID #8); und
einem zweiten Satz, umfassend:
einen ersten Primer, umfassend die Sequenz CATCCTGCTGCTCTCGGGAG (SEQ ID #30); und
einen zweiten Primer, umfassend die Sequenz CCACAGCTCCTAGGACAGCTAGGA (SEQ ID #8).

27. Ein Sequenzierprimer zur Sequenzierung des Exon 2 der HLA-A, -B, oder -C Klasse I Gene, umfassend eine Sequenz, ausgewählt aus der Gruppe bestehend aus der Sequenz CTCGCCCCCAGGCTCCCAC (SEQ ID #9); der Sequenz CACTCACCGGCCTCGCTCTGG (SEQ ID #12); aus den degenerierten Sequenzen TCGGGCAGGTCTCAGCC (SEQ ID #25) und TCGGGCGGGTCTCAGCC (SEQ ID #26); und aus der Sequenz AGGAGGGTCGGGCGGGTCTCAG (SEQ ID #31).

28. Ein Sequenzierprimer zur Sequenzierung des Exon 3 der HLA-A, -B, oder -C Klasse 1 Gene, umfassend eine Sequenz, ausgewählt aus der Gruppe bestehend aus der Sequenz GCGGGGGCGGGTCCAGG (SEQ ID #10); aus der Sequenz CCACTGCCCCTGGTACCCG (SEQ ID #13); der Sequenz GAGGCGCCCCGTGGC (SEQ ID #29); aus den degenerierten Sequenzen GGGCTCGGGGGACCGGG (SEQ ID #27) und GGGCTCGGGGGACTGGG (SEQ ID #28); und der Sequenz GGGCTGACCACGGGGGCGGGGCCCAG (SEQ ID #32).

29. Ein Sequenzierprimer zur Sequenzierung des Exon 4 der HLA-A, -B, oder -C Klasse I Gene, umfassend eine Sequenz, ausgewählt aus der Gruppe bestehend aus der Sequenz CTGACTCTTCCCATCAGACCC (SEQ ID #11); und der Sequenz AGGGTGAGGGGCTTCGGCAGCC (SEQ ID #14).

## Revendications

1. Procédé utile pour le typage de gènes HLA-A, -B ou -C de classe I, comprenant les étapes de:
fourniture d'un ADN échantillon contenant un gène HLA-A, -B ou -C de classe 1 ayant un premier exon, un second exon et une séquence cible;
la mise en contact de l'ADN échantillon avec une première amorce d'amplification, la première amorce d'amplification comportant une séquence complémentaire de la séquence située dans l'exon 1 du gène HLA-A, -B ou -C;
la mise en contact de l'ADN échantillon avec une seconde amorce d'amplification, la seconde amorce d'amplification comportant une séquence complémentaire de la séquence située dans l'exon 5 du gène HLA-A, -B ou -C;
l'amplification de la séquence cible de l'ADN échantillon par ACP en utilisant les première et seconde amorces d'amplification, de façon à former un amplicon; et
la détection de l'amplicon en utilisant une méthode de détection spécifique des séquences.

2. Procédé selon la revendication 1, dans lequel le gène HLA de classe I est un gène HLA-A et la première amorce d'amplification comprend la séquence CGCCGAGGATGGCCGTC (SEQ ID NO 1) et la seconde amorce d'amplification comprend les séquences dégénérées GGAGAACCAGGCCAGCAATGATGCCC (SEQ ID NO 2) et GGAGAACTAGGCCAGCAATGATGCCC (SEQ ID NO 3).

3. Procédé selon la revendication 1, dans lequel le gène HLA de classe I est un gène HLA-B et la première amorce d'amplification comprend les séquences dégénérées CCTCCTGCTGCTCTCGGC (SEQ ID NO 21) et CCTCCTGCTGCTCTCGGGA (SEQ ID NO 22) et GCTGCTCTGGGGGGCAG (SEQ ID NO 23) et la seconde amorce d'amplification comprend la séquence GCTCCGATGACCACAACTGCT (SEQ ID NO 24).

4. Procédé selon la revendication 1, dans lequel le gène HLA de classe I est un gène HLA-B et la première amorce d'amplification comprend la séquence GGCCCTGACCGAGACCTGG (SEQ ID NO.4) et la deuxième amorce d'amplification comprend la séquence TCCGATGACCACAACTGCTAGGAC (SEQ ID NO.5).

5. Procédé selon la revendication 1, dans lequel le gène HLA de classe I est un gène HLA-C et la première amorce d'amplification comprend les séquences dégénérées GGCCCTGACCGAGACCTGGGC (SEQ ID NO.6) et GGCCCTGACCCAGACCTGGGC (SEQ ID NO.7) et la deuxième amorce d'amplification comprend la séquence CCACAGCTCCTAGGACAGCTAGGA (SEQ ID NO.8).

6. Procédé selon la revendication 1, dans lequel le gène HLA de classe I est un gène HLA-C et la première amorce d'amplification comprend la séquence CATCCTGCTGCTCTCGGGAG (SEQ ID NO.30) et la deuxième amorce d'amplification comprend la séquence CCACAGCTCCTAGGACAGCTAGGA (SEQ ID NO.8).

7. Procédé selon la revendication 1, dans lequel la méthode de détection spécifique d'une séquence est le séquençage d'ADN.

8. Procédé selon la revendication 7, dans lequel la méthode de séquençage d'ADN est un séquençage d'ADN de type Sanger comprenant les étapes de:
mise en contact de l'amplicon avec une amorce de séquençage,
extension de l'amorce de séquençage en utilisant une polymérase en présence de désoxynucléotides et de didésoxynucléotides, formant ainsi un mélange de produits d'extension différents dans leur longueur,
séparation des produits d'extension de telle sorte que les produits qui différent dans leur longueur par un seul nucléotide sont séparés, et
détection des produits d'extension séparés.

9. Procédé selon la revendication 8, dans lequel l'amorce de séquençage (i) a une longueur entre 10 nucléotides et 30 nucléotides et (ii) a une séquence nucléotidique complémentaire des gènes HLA-A, HLA-B et HLA-C dans les régions de séquence nucléotidique choisies dans le groupe de:
une première région de séquence nucléotidique allant de -20 nucléotides à +20 nucléotides de la limite de 5' intron-exon du brin sens de l'exon 2,
une deuxième région de séquence nucléotidique allant de -30 nucléotides à +20 nucléotides de la limite de 5' intron-exon du brin sens de l'exon 3,
une troisième région de séquence nucléotidique allant de -30 nucléotides à +20 nucléotides de la limite de 5' intron-exon du brin sens de l'exon 4,
une quatrième région de séquence nucléotidique allant de +30 nucléotides à -20 nucléotides de la limite de 5' intron-exon du brin antisens de l'exon 2,
une cinquième région de séquence nucléotidique allant de +20 nucléotides à -20 nucléotides de la limite de 5' intron-exon du brin antisens de l'exon 3, et
une sixième région de séquence nucléotidique allant de +40 nucléotides à -10 nucléotides de la limite de 5' intron-exon du brin antisens de l'exon 4.

10. Procédé selon la revendication 9, dans lequel chaque membre de la série d'amorces de séquençage est choisi dans le groupe consistant en:
une première amorce de séquençage ayant la séquence CTCGCCCCCAGGCTCCCAC (SEQ ID NO.15),
une deuxième amorce de séquençage ayant la séquence GCGGGGGCGGGTCCAGG (SEQ ID NO.16),
une troisième amorce de séquençage ayant la séquence CTGACTCTTCCCATCAGACCC (SEQ ID NO.17),
une quatrième amorce de séquençage ayant la séquence CACTCACCGGCCTCGCTCTGG (SEQ ID NO.18),
une cinquième amorce de séquençage ayant la séquence CCACTGCCCCTGGTACCCG (SEQ ID NO.19), et
une sixième amorce de séquençage ayant la séquence AGGGTGAGGGGCTTCGGCAGCC (SEQ ID NO.20).

11. Procédé selon la revendication 8, dans lequel le gène HLA de classe 1 est le brin sens de l'exon 2 du gène HLA-A et l'amorce de séquençage comprend les séquences dégénérées TCGGGCAGGTCTCAGCC (SEQ ID NO.25) et TCGGGCGGGTCTCAGCC (SEQ ID NO.26).

12. Procédé selon la revendication 8, dans lequel le gène HLA de classe 1 est le brin antisens de l'exon 2 du gène HLA-A et l'amorce de séquençage comprend la séquence CACTCACCGGCCTCGCTCTGG (SEQ ID NO.12).

13. Procédé selon la revendication 8, dans lequel le gène HLA de classe 1 est le brin sens de l'exon 3 du gène HLA-A et l'amorce de séquençage comprend les séquences dégénérées GGGCTCGGGGGACCGGG (SEQ ID NO.27) et GGGCTCGGGGGACTGGG (SEQ ID NO.28).

14. Procédé selon la revendication 8, dans lequel le gène HLA de classe 1 est le brin sens de l'exon 2 du gène HLA-B et l'amorce de séquençage comprend la séquence CTCGCCCCCAGGCTCCCAC (SEQ ID NO.9).

15. Procédé selon la revendication 8, dans lequel le gène HLA de classe 1 est le brin antisens de l'exon 2 du gène HLA-B et l'amorce de séquençage comprend la séquence CACTCACCGGCCTCGCTCTGG (SEQ ID NO.12).

16. Procédé selon la revendication 8, dans lequel le gène HLA de classe 1 est le brin sens de l'exon 3 du gène HLA-B et l'amorce de séquençage comprend la séquence GCGGGGGCGGGTCCAGG (SEQ ID NO.10).

17. Procédé selon la revendication 8, dans lequel le gène HLA de classe 1 est le brin antisens de l'exon 3 du gène HLA-B et l'amorce de séquençage comprend la séquence CCACTGCCCCTGGTACCCG (SEQ ID NO.13).

18. Procédé selon la revendication 8, dans lequel le gène HLA de classe 1 est le brin sens de l'exon 2 du gène HLA-C et l'amorce de séquençage comprend la séquence AGGAGGGTCGGGCGGGTCTCAG (SEQ ID NO.31).

19. Procédé selon la revendication 8, dans lequel le gène HLA de classe 1 est le brin antisens de l'exon 2 du gène HLA-C et l'amorce de séquençage comprend la séquence CACTCACCGGCCTCGCTCTGG (SEQ ID NO.12).

20. Procédé selon la revendication 8, dans lequel le gène HLA de classe 1 est le brin antisens de l'exon 3 du gène HLA-C et l'amorce de séquençage comprend la séquence CCACTGCCCCTGGTACCCG (SEQ ID NO.13).

21. Procédé selon la revendication 1, comprenant en outre l'étape de comparaison de la séquence d'ADN déterminée avec les séquences d'ADN de types de HLA connus.

22. Kit ou ensemble utile pour le typage de gènes HLA-A, -B ou -C de classe I comprenant:
une polymérase thermostable,
chacun des désoxynucléotides A, G, C et T,
un tampon, et
une première et une deuxième amorces d'amplification, chaque amorce d'amplification étant ayant une longueur entre 10 et 30 nucléotides, la première amorce ayant une séquence nucléotidique complémentaire de la séquence située dans l'exon 1 du gène HLA-A, -B ou -C, et la deuxième amorce ayant une séquence nucléotidique complémentaire de la séquence située dans l'exon 5 du gène HLA-A, -B ou -C.

23. Kit selon la revendication 22, comprenant en outre:
un réactif de séquençage comprenant:
une polymérase,
chacun des désoxynucléotides A, G, C et T,
chacun des didésoxynucléotides A, G, C et T,
un tampon, et
et une série d'amorces de séquençage, dans laquelle chaque membre de la série d'amorces de séquençage (i) a une longueur entre 10 nucléotides et 30 nucléotides et (ii) a une séquence nucléotidique complémentaire des gènes HLA-A, HLA-B et HLA-C dans les régions de séquence nucléotidique choisies dans le groupe de:
une première région de séquence nucléotidique allant de -20 à +20 nucléotides de la limite de 5' intron-exon du brin sens de l'exon 2,
une deuxième région de séquence nucléotidique allant de -30 à +20 nucléotides de la limite de 5' intron-exon du brin sens de l'exon 3,
une troisième région de séquence nucléotidique allant de -30 à +20 nucléotides de la limite de 5' intron-exon du brin sens de l'exon 4,
une quatrième région de séquence nucléotidique allant de +30 à -20 nucléotides de la limite de 5' intron-exon du brin antisens de l'exon 2,
une cinquième région de séquence nucléotidique allant de +20 à -20 nucléotides de la limite de 5' intron-exon du brin antisens de l'exon 3, et
une sixième région de séquence nucléotidique allant de +40 à-10 nucléotides de la limite de 5' intron-exon du brin antisens de l'exon 4.

24. Amorces d'amplification pour ACP utiles pour l'amplification du gène HLA-A de classe 1, comprenant:
une première amorce comprenant la séquence CGCCGAGGATGGCCGTC (SEQ ID NO.1), et
une deuxième amorce comprenant les séquences dégénérées GGAGAACCAGGCCAGCAATGATGCCC (SEQ ID NO.2) et GGAGAACTAGGCCAGCAATGATGCCC (SEQ ID NO.3).

25. Amorces d'amplification pour ACP utiles pour l'amplification du gène HLA-B de classe 1, choisies dans le groupe consistant en:
une première série comprenant
une première amorce comprenant la séquence GGCCCTGACCGAGACCTGG (SEQ ID NO.4), et
une deuxième amorce comprenant la séquence TCCGATGACCACAACTGCTAGGAC (SEQ ID NO.5), et
une deuxième série comprenant:
une première amorce comprenant les séquences dégénérées CCTCCTGCTGCTCTCGGC (SEQ ID NO.21), et CCTCCTGCTGCTCTCGGGA (SEQ ID NO.22), et GCTGCTCTGGGGGGCAG (SEQ ID NO.23) ; et
une deuxième amorce comprenant la séquence GCTCCGATGACCACAACTGCT (SEQ ID NO.24).

26. Amorces d'amplification pour ACP utiles pour l'amplification du gène HLA-C de classe 1 choisies dans le groupe consistant en:
une première série comprenant
une première amorce comprenant les séquences dégénérées GGCCCTGACCGAGACCTGGGC (SEQ ID NO.6) et GGCCCTGACCCAGACCTGGGC (SEQ ID NO.7), et
une deuxième amorce comprenant la séquence CCACAGCTCCTAGGACAGCTAGGA (SEQ ID NO.8), et
une deuxième série comprenant:
une première amorce comprenant la séquence CATCCTGCTGCTCTCGGGAG (SEQ ID NO.30), et
une deuxième amorce comprenant la séquence CCACAGCTCCTAGGACAGCTAGGA (SEQ ID NO.8).

27. Amorce de séquençage utile pour le séquençage d'exon 2 de gènes HLA-A, -B ou -C de classe 1,comprenant une séquence choisie dans le groupe consistant en la séquence CTCGCCCCCAGGCTCCCAC (SEQ ID NO.9), la séquence CACTCACCGGCCTCGCTCTGG (SEQ ID NO.12), les séquences dégénérées TCGGGCAGGTCTCAGCC (SEQ ID NO.25) et TCGGGCGGGTCTCAGCC SEQ ID NO.26), et la séquence AGGAGGGTCGGGCGGGTCTCAG (SEQ ID NO.31).

28. Amorce de séquençage utile pour le séquençage d'exon 3 de gènes HLA-A, -B ou -C de classe 1,comprenant une séquence choisie dans le groupe consistant en la séquence GCGGGGGCGGGTCCAGG (SEQ ID NO.10), la séquence CCACTGCCCCTGGTACCCG (SEQ ID NO.13), la séquence GAGGCGCCCCGTGGC (SEQ ID NO.29), les séquences dégénérées GGGCTCGGGGGACCGGG (SEQ ID NO.27) et GGGCTCGGGGGACTGGG (SEQ ID NO.28), et la séquence GGGCTGACCACGGGGGCGGGGCCCAG (SEQ ID NO.32).

29. Amorce de séquençage utile pour le séquençage d'exon 4 de gènes HLA-A, -B ou -C de classe 1,comprenant une séquence choisie dans le groupe consistant en la séquence CTGACTCTTCCCATCAGACCC (SEQ ID NO.11) et la séquence AGGGTGAGGGGCTTCGGCAGCC (SEQ ID NO.14).


### SEQUENCE LISTING

(1) GENERAL INFORMATION:
   (i) APPLICANT:
   (ii) TITLE OF INVENTION: Method and reagents for typing HLA class I genes
   (iii) NUMBER OF SEQUENCES: 32
   (iv) COMPUTER READABLE FORM:
      (A) MEDIUM TYPE: 3.5 inch diskette
      (B) COMPUTER: IBM compatible
      (C) OPERATING SYSTEM: Windows 3.10/DOS 6.20
      (D) SOFTWARE: Microsoft Word for Windows, vers. 6.0
   (v) CURRENT APPLICATION DATA:
      (A) APPLICATION NUMBER: EP 96 907 091.1
      (B) FILING DATE: 20 FEBRUARY 1996
      (C) CLASSIFICATION:
   (vi) PRIOR APPLICATION DATA:
      (A) APPLICATION NUMBER:
      (B) FILING DATE:
(2) INFORMATION FOR SEQ ID NO: 1:
   (i) SEQUENCE CHARACTERISTICS:
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 1:
   CGCCGAGGAT GGCCGTC                                                  17
(2) INFORMATION FOR SEQ ID NO: 2:
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (xi) SEQUENCE DESCRIPTION: SEQ ID NO: 2:
   GGAGAACCAG GCCAGCAATG ATGCCC 26
(2) INFORMATION FOR SEQ ID NO: 3:
   (i) SEQUENCE CHARACTERISTICS:
      (A) LENGTH: 26 nucleotides
      (B) TYPE: nucleic acid
      (C) STRANDEDNESS: single
      (D) TOPOLOGY: linear
   (xi) SEQUENCE DESCRIPTION: SEQ ID NO: 3:
   GGAGAACTAG GCCAGCAATG ATGCCC 26
(2) INFORMATION FOR SEQ ID NO: 4:
   (i) SEQUENCE CHARACTERISTICS:
      (A) LENGTH: 19 nucleotides
      (B) TYPE: nucleic acid
      (C) STRANDEDNESS: single
      (D) TOPOLOGY: linear
   (xi) SEQUENCE DESCRIPTION: SEQ ID NO: 4:
   GGCCCTGACC GAGACCTGG 19
(2) INFORMATION FOR SEQ ID NO: 5:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (xi) SEQUENCE DESCRIPTION: SEQ ID NO: 5:
   TCCGATGACC ACAACTGCTA GGAC 24
(2) INFORMATION FOR SEQ ID NO: 6:
   (i) SEQUENCE CHARACTERISTICS:
      (A) LENGTH: 21 nucleotides
      (B) TYPE: nucleic acid
      (C) STRANDEDNESS: single
      (D) TOPOLOGY: linear
   (xi) SEQUENCE DESCRIPTION: SEQ ID NO: 6:
(2) INFORMATION FOR SEQ ID NO: 7:
   (i) SEQUENCE CHARACTERISTICS:
      (A) LENGTH: 21 nucleotides
      (B) TYPE: nucleic acid
      (C) STRANDEDNESS: single
      (D) TOPOLOGY: linear
   (xi) SEQUENCE DESCRIPTION: SEQ ID NO: 7:
   GGCCCTGACC CAGACCTGGG C 21
(2) INFORMATION FOR SEQ ID NO: 8:
   (i) SEQUENCE CHARACTERISTICS:
      (A) LENGTH: 24 nucleotides
      (B) TYPE: nucleic acid
      (C) STRANDEDNESS: single
      (D) TOPOLOGY: linear
   (xi) SEQUENCE DESCRIPTION: SEQ ID NO: 8:
(2) INFORMATION FOR SEQ ID NO: 9:
   (i) SEQUENCE CHARACTERISTICS:
      (A) LENGTH: 19 nucleotides
      (B) TYPE: nucleic acid
      (C) STRANDEDNESS: single
      (D) TOPOLOGY: linear
   (xi) SEQUENCE DESCRIPTION: SEQ ID NO: 9:
   CTCGCCCCCA GGCTCCCAC 19
(2) INFORMATION FOR SEQ ID NO: 10:
   (i) SEQUENCE CHARACTERISTICS:
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 10:
   GCGGGGGCGG GTCCAGG 17
(2) INFORMATION FOR SEQ ID NO: 11:
   (i) SEQUENCE CHARACTERISTICS:
      (A) LENGTH: 21 nucleotides
      (B) TYPE: nucleic acid
      (C) STRANDEDNESS: single
      (D) TOPOLOGY: linear
   (xi) SEQUENCE DESCRIPTION: SEQ ID NO: 11:
   CTGACTCTTC CCATCAGACC C 21
(2) INFORMATION FOR SEQ ID NO: 12:
   (i) SEQUENCE CHARACTERISTICS:
      (A) LENGTH: 21 nucleotides
      (B) TYPE: nucleic acid
      (C) STRANDEDNESS: single
      (D) TOPOLOGY: linear
   (xi) SEQUENCE DESCRIPTION: SEQ ID NO: 12:
   CACTCACCGG CCTCGCTCTG G 21
(2) INFORMATION FOR SEQ ID NO: 13:
   (i) SEQUENCE CHARACTERISTICS
      (A) LENGTH: 19 nucleotides
      (B) TYPE: nucleic acid
      (C) STRANDEDNESS: single
      (D) TOPOLOGY: linear
   (xi) SEQUENCE DESCRIPTION: SEQ ID NO: 13:
   CCACTGCCCC TGGTACCCG 19
(2) INFORMATION FOR SEQ ID NO: 14
   (i) SEQUENCE CHARACTERISTICS
      (A) LENGTH: 22 nucleotides
      (B) TYPE: nucleic acid
      (C) STRANDEDNESS: single
      (D) TOPOLOGY: linear
   (xi) SEQUENCE DESCRIPTION: SEQ ID NO: 14:
   AGGGTGAGGG GCTTCGGCAG CC 22
(2) INFORMATION FOR SEQ ID NO: 15
   (i) SEQUENCE CHARACTERISTICS
      (A) LENGTH: 19 nucleotides
      (B) TYPE: nucleic acid
      (C) STRANDEDNESS: single
      (D) TOPOLOGY: linear
   (xi) SEQUENCE DESCRIPTION: SEQ ID NO: 15:
   CTCGCCCCCA GGCTCCCAC 19
(2) INFORMATION FOR SEQ ID NO: 16
   (i) SEQUENCE CHARACTERISTICS
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 16:
   GCGGGGGCGG GTCCAGG 17
(2) INFORMATION FOR SEQ ID NO: 17
   (i) SEQUENCE CHARACTERISTICS
      (A) LENGTH: 21 nucleotides
      (B) TYPE: nucleic acid
      (C) STRANDEDNESS: single
      (D) TOPOLOGY: linear
   (xi) SEQUENCE DESCRIPTION: SEQ ID NO: 17:
   CTGACTCTTC CCATCAGACC C 21
(2) INFORMATION FOR SEQ ID NO: 18
   (i) SEQUENCE CHARACTERISTICS
      (A) LENGTH: 21 nucleotides
      (B) TYPE: nucleic acid
      (C) STRANDEDNESS: single
      (D) TOPOLOGY: linear
   (xi) SEQUENCE DESCRIPTION: SEQ ID NO: 18:
   CACTCACCGG CCTCGCTCTG G 21
(2) INFORMATION FOR SEQ ID NO: 19
   (i) SEQUENCE CHARACTERISTICS
      (A) LENGTH: 19 nucleotides
      (B) TYPE: nucleic acid
      (C) STRANDEDNESS: single
      (D) TOPOLOGY: linear
   (xi) SEQUENCE DESCRIPTION: SEQ ID NO: 19:
   CCACTGCCCC TGGTACCCG 19
(2) INFORMATION FOR SEQ ID NO: 20
   (i) SEQUENCE CHARACTERISTICS
      (A) LENGTH: 22 nucleotides
      (B) TYPE: nucleic acid
      (C) STRANDEDNESS: single
      (D) TOPOLOGY: linear
   (xi) SEQUENCE DESCRIPTION: SEQ ID NO: 20:
   AGGGTGAGGG GCTTCGGCAG CC 22
2) INFORMATION FOR SEQ ID NO: 21
   (i) SEQUENCE CHARACTERISTICS
      (A) LENGTH: 18 nucleotides
      (B) TYPE: nucleic acid
      (C) STRANDEDNESS: single
      (D) TOPOLOGY: linear
   (xi) SEQUENCE DESCRIPTION: SEQ ID NO: 21:
   CCTCCTGCTG CTCTCGGC 18
2) INFORMATION FOR SEQ ID NO: 22
   (i) SEQUENCE CHARACTERISTICS
      (A) LENGTH: 19 nucleotides
      (B) TYPE: nucleic acid
      (C) STRANDEDNESS: single
      (D) TOPOLOGY: linear
   (xi) SEQUENCE DESCRIPTION: SEQ ID NO: 22
   CCTCCTGCTG CTCTCGGGA 19
2) INFORMATION FOR SEQ ID NO: 23
   (i) SEQUENCE CHARACTERISTICS
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 23:
   GCTGCTCTGG GGGGCAG 17
2) INFORMATION FOR SEQ ID NO: 24
   (i) SEQUENCE CHARACTERISTICS
      (A) LENGTH: 21 nucleotides
      (B) TYPE: nucleic acid
      (C) STRANDEDNESS: single
      (D) TOPOLOGY: linear
   (xi) SEQUENCE DESCRIPTION: SEQ ID NO: 24:
2) INFORMATION FOR SEQ ID NO: 25
   (i) SEQUENCE CHARACTERISTICS
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 25
   TCGGGCAGGT CTCAGCC 17
2) INFORMATION FOR SEQ ID NO: 26
   (i) SEQUENCE CHARACTERISTICS
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 26:
   TCGGGCGGGTCTCAGCC 17
2) INFORMATION FOR SEQ ID NO: 27
   (i) SEQUENCE CHARACTERISTICS
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 27:
   GGGCTCGGGG GACCGGG 17
2) INFORMATION FOR SEQ ID NO: 28
   (i) SEQUENCE CHARACTERISTICS
      (A) LENGTH: 17 nucleotides
      (B) TYPE: nucleic acid
      (C) STRANDEDNESS: single
      (D) TOPOLOGY: linear
   (xi) SEQUENCE DESCRIPTION: SEQ ID NO: 28:
   GGGCTCGGGG GACTGGG 17
2) INFORMATION FOR SEQ ID NO: 29
   (i) SEQUENCE CHARACTERISTICS
      (A) LENGTH: 15 nucleotides
      (B) TYPE: nucleic acid
      (C) STRANDEDNESS: single
      (D) TOPOLOGY: linear
   (xi) SEQUENCE DESCRIPTION: SEQ ID NO: 29:
   GAGGCGCCCC GTGGC 15
2) INFORMATION FOR SEQ ID NO: 30
   (i) SEQUENCE CHARACTERISTICS
      (A) LENGTH: 20 nucleotides
      (B) TYPE: nucleic acid
      (C) STRANDEDNESS: single
      (D) TOPOLOGY: linear
   (xi) SEQUENCE DESCRIPTION: SEQ ID NO: 30:
   CATCCTGCTG CTCTCGGGAG 20
2) INFORMATION FOR SEQ ID NO: 31
   (i) SEQUENCE CHARACTERISTICS
      (A) LENGTH: 22 nucleotides
      (B) TYPE: nucleic acid
      (C) STRANDEDNESS: single
      (D) TOPOLOGY: linear
   (xi) SEQUENCE DESCRIPTION: SEQ ID NO: 31:
2) INFORMATION FOR SEQ ID NO: 32
   (i) SEQUENCE CHARACTERISTICS
      (A) LENGTH: 26 nucleotides
      (B) TYPE: nucleic acid
      (C) STRANDEDNESS: single
      (D) TOPOLOGY: linear
   (xi) SEQUENCE DESCRIPTION: SEQ ID NO: 32:
   GGGCTGACCA CGGGGGCGGG GCCCAG 26